# EUROPEAN PATENT SPECIFICATION

(11) **EP 2 046 765 B1**
(45) Date of publication and mention of the grant of the patent: **17.10.2012**
(21) Application number: 07786198.7
(22) Date of filing: 19.07.2007
(51) Int. Cl.: C07D 261/04, C07D 413/12, C07D 417/12, A01N 43/80, C07C 237/42, C07C 211/52

(54) **INSECTICIDAL COMPOUNDS**
INSEKTIZIDE VERBINDUNGEN
COMPOSÉS INSECTICIDES

(30) Priority: 24.07.2006 GB 0614691
(43) Date of publication of application: 15.04.2009
(73) Proprietor: Syngenta Participations AG, 4058 Basel (CH)
(72) Inventor: RENOLD, Peter, CH-4332 Stein (CH); MAIENFISCH, Peter, CH-4332 Stein (CH); JUNG, Pierre, CH-4332 Stein (CH); LUTZ, William, CH-4125 Riehen (CH); ZAMBACH, Werner, CH-4332 Stein (CH)
(74) Representative: Ward, Steven Paul
(86) International application number: PCT/EP2007/006431
(87) International publication number: WO 2008/012027

(56) References cited:
- WO-A-2005/073165
- US-A- 4 008 275
- US-A- 4 316 850
- US-A- 4 587 361
- US-A1- 2005 250 822
- DATABASE CA [Online] CHEMICAL ABSTRACTS SERVICE, COLUMBUS, OHIO, US; JIANG, BIAO ET AL: "Preparation of 1-Trifluoromethyl-2-alkylvinylaniline derivatives" XP002454721 retrieved from STN Database accession no. 2001:174453 & CN 1 263 084 A (SHANGHAI INST. OF ORGANIC CHEMISTRY, CHINESE ACADEMY OF SCIENCES, PEOP) 16 August 2000 (2000-08-16)

## Description

The present invention relates to certain aromatic bisamide derivatives, to processes and intermediates for preparing them, to insecticidal, acaricidal, molluscicidal and nematicidal compositions comprising them and to methods of using them to combat and control insect, acarine, mollusc and nematode pests.

Aromatic bisamide derivatives with insecticidal properties are disclosed, for example, in EP 1,714,958, JP 2006/306771, WO 06/137376, WO 06/137395 WO 07/017075 and WO 2005/073165.

It has now surprisingly been found that certain aromatic bisamide derivatives which are substituted by an isoxazolinyl substituent have insecticidal properties.

The present invention therefore provides a compound of formula (I): 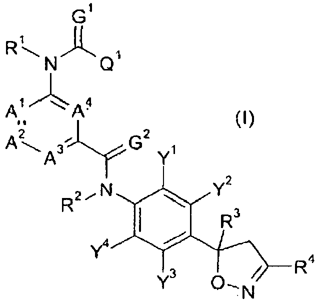 wherein
A¹, A², A³ and A⁴ are C-X;
each X is independently hydrogen, halogen, C₁-C₄alkyl, C₁-C₄haloalkyl or C₁-C₄alkoxy; R¹ and R² are hydrogen;
G¹ and G² are oxygen;
Q¹ is phenyl or phenyl substituted by one to five substituents R⁵, which may be the same or different, or Q¹ is heterocyclyl or heterocyclyl substituted by one to five substituents R⁵, which may be the same or different;
R³ is trifluoromethyl;
R⁴ is phenyl or phenyl substituted by one to five substituents R⁵, which may be the same or different;
Y¹ and Y⁴ are independently of each other hydrogen, cyano, halogen, C₁-C₄alkyl or C₁-C₄haloalkyl;
Y² and Y³ are independently of each other hydrogen, halogen or C₁-C₄alkyl; and each R⁵ is independently cyano, nitro, hydroxy, halogen, C₁-C₄alkyl, C₁-C₄haloatkyl, C₁-C₃alkoxy or C₁-C₃alkylthio;
wherein said heterocyclyl is selected from pyridyl, pyridazinyl, pyrimidinyl, pyrazinyl, furanyl, thiophenyl, oxazolyl, isoxazolyl, oxadiazolyl, thiazolyl, isothiazolyl, thiadiazolyl, pyrrolyl, pyrazolyl, imidazolyl, triazolyl and tetrazolyl;
or salts or N-oxides thereof.

The compounds of formula (I) may exist in different geometric or optical isomers or tautomeric forms. This invention covers all such isomers and tautomers and mixtures thereof in all proportions as well as isotopic forms such as deuterated compounds.

Each alkyl moiety either alone or as part of a larger group (such as alkoxy, alkoxycarbonyl, alkylcarbonyl) is a straight or branched chain and is, for example, methyl, ethyl, n-propyl, n-butyl, iso-propyl, *n*-butyl, sec-butyl, iso-butyl or tert-butyl. The alkyl groups are preferably C₁ to C₆ alkyl groups, more preferably C₁-C₄ and most preferably C₁-C₃ alkyl groups.

Alkenyl and alkynyl moieties (either alone or as part of a larger group, such as alkenyloxy or alkynyloxy) can be in the form of straight or branched chains, and the alkenyl moieties, where appropriate, can be of either the (E)- or (Z)-configuration. Examples are vinyl, allyl and propargyl. The alkenyl and alkynyl groups are preferably C₂ to C₆ alkenyl or alkynyl groups, more preferably C₂-C₄ and most preferably C₂-C₃ alkenyl or alkynyl groups.

Halogen is fluorine, chlorine, bromine or iodine.

Haloalkyl groups (either alone or as part of a larger group, such as haloalkoxy or haloalkylthio) are alkyl groups which are substituted with one or more of the same or different halogen atoms and are, for example, -CF₃, -CF₂Cl, -CH₂CF₃ or -CH₂CHF₂. Perfluoroalkyl groups (either alone or as part of a larger group, such as perfluoroalkylthio) are a particular type of haloalkyl group; they are alkyl groups which are completely substituted with fluorine atoms and are, for example, -CF₃, -CF₂CF₃ or -CF(CF₃)₂.

Haloalkenyl and haloalkynyl groups (either alone or as part of a larger group, such as haloalkenyloxy or haloalkynyloxy) are alkenyl and alkynyl groups, respectively, which are substituted with one or more of the same or different halogen atoms and are, for example, -CH=CF₂, -CCl=CClF or -CHClC≡CH.

Cycloalkyl groups can be in mono- or bi-cyclic form and may optionally be substituted by one or more methyl groups. The cycloalkyl groups preferably contain 3 to 8 carbon atoms, more preferably 3 to 6 carbon atoms. Examples of monocyclic cycloalkyl groups are cyclopropyl, 1-methylcyclopropyl, 2-methylcyclopropyl, cyclobutyl, cyclopentyl and cyclohexyl.

Halocycloalkyl groups are cycloalkyl groups which are substituted with one or more of the same of different halogen atoms and may optionally be substituted by one or more methyl groups. Examples of monocyclic halocycloalkyl groups are 2,2-dichloro-cyclopropyl, 2,2-dichloro-1-methyl-cyclopropyl and 2-chloro-4-fluorocyclohexyl.

In the context of the present specification the term "aryl" refers to a ring system which may be mono-, bi- or tricyclic. Examples of such rings include phenyl, naphthalenyl, anthracenyl, indenyl or phenanthrenyl. A preferred aryl group is phenyl.

The term "heteroaryl" refers to an aromatic ring system containing at least one heteroatom and consisting either of a single ring or of two or more fused rings. Preferably, single rings will contain up to three and bicyclic systems up to four heteroatoms which will preferably be chosen from nitrogen, oxygen and sulfur. Examples of such groups include pyridyl, pyridazinyl, pyrimidinyl, pyrazinyl, furanyl, thiophenyl, oxazolyl, isoxazolyl, oxadiazolyl, thiazolyl, isothiazolyl, thiadiazolyl, pyrrolyl, pyrazolyl, imidazolyl, triazolyl and tetrazolyl. A preferred heteroaryl group is pyridine. Examples of bicyclic groups are benzothiophenyl, benzimidazolyl, benzothiadiazolyl, quinolinyl, cinnolinyl and quinoxalinyl.

The term "heterocyclyl" is defined to include heteroaryl and in addition their unsaturated or partial ly unsaturated analogues such as 4,5,6,7 -tetrahydro-benzothiophenyl, 9H-fluorenyl, 3,4-dihydro-2H-benzo-1,4-dioxepinyl, 2,3-dihydro-benzofuranyl, piperidinyl, 1,3-dioxolanyl, 1,3-dioxanyl, 4,5-dihydro-isoxazolyl, tetrahydrofuranyl and morpholinyl.

Preferred values of A¹, A², A³, A⁴, X, R¹, R², G¹, G², Q¹, R³, R⁴, Y¹, Y², Y³ and Y⁴ are, in any combination, as set out below.

Preferably each X is independently hydrogen, fluoro, chloro, bromo, methyl, trifluoromethyl or methoxy.

More preferably each X is independently hydrogen, fluoro, chloro, bromo, methyl or trifluoromethyl.

Even more preferably each X is independently hydrogen, fluoro, methyl or trifluoromethyl.

Yet even more preferably each X is independently hydrogen or fluoro.

Most preferably each X is hydrogen.

Preferably Q¹ is phenyl, pyridyl, furanyl, thiophenyl, pyrazolyl or 1,2,3-thiadiazolyl, or phenyl, pyridyl, furanyl, thiophenyl, pyrazolyl or 1,2,3-thiadiazolyl substituted by one to three substituents independently selected from cyano, nitro, hydroxy, fluoro, chloro, bromo, methyl, trifluoromethyl, methoxy or methylthio. Examples of such groups for Q¹ are 5-bromo-furan-2-yl, 2-bromo-phenyl, 5-bromo-pyrid-3-yl, 2-chloro-5-nitro-phenyl, 2-chloro-phenyl, 3-chloro-phenyl, 2-chloro-pyrid-3-yl, 2-chloropyrid-4-yl, 6-chloro-pyrid-3-yl, 5-chloro-thiophen-2-yl, 3-chloro-5-trifluoromethyl-pyrid-2-yl, 4-cyano-phenyl, 2,5-dichloro-phenyl, 2,3-difluoro-phenyl, 1,3-dimethyl-1H-pyrazol-5-yl, 4-fluoro-phenyl, 2-fluoro-pyrid-3-yl, 2-fluoro-3-trifluoromethyl-phenyl, 2-methyl-phenyl, 3-methyl-pyrid-2-yl, 2-methylthio-pyrid-3-yi, 4-nitro-phenyl, phenyl, 1,2,3-thiadiazol-4-yl, thiophen-2-yl and 4-trifluoromethyl-phenyl.

More preferably Q¹ is phenyl or pyridyl, or phenyl or pyridyl substituted by one to three substituents independently selected from cyano, hydroxy, fluoro, chloro, methyl, trifluoromethyl, methoxy: or methylthio. Examples of more preferred groups for Q¹ are 2-chloro-phenyl, 3-chloro-phenyl, 2-chloro-pyrid-3-yl, 2-chloro-pyrid-4-yl, 6-chloro-pyrid-3-yl, 3-chloro-5-trifluoromethyl-pyrid-2-yl, 4-cyano-phenyl, 2,5-dichloro-phenyl, 2,3-difluoro-phenyl, 4-tluoro-phenyl, 2-fluoro-pyrid-3-yl, 2-fluoro-3-trifluoromethyl-phenyl, 2-methyl-phenyl, 3-methyl-pyrid-2-yl, 2-methylthio-pyrid-3-yl, phenyl and 4-tritluoromethyl-phenyl.

Even more preferably Q¹ is phenyl substituted by one substituent selected from cyano, fluoro or chloro. Examples of even more preferred groups for Q¹ are 2-chloro-phenyl, 3-chloro-phenyl, 4-cyano-phenyl and 4-fluoro-phenyl.

Most preferably Q¹ is 4-fluoro-phenyl.

Preferably R⁴ is phenyl or phenyl substituted by one to five substituents selected from halogen, C₁-C₄alkyl, C₁-C₄haloalkyl.

Even more preferably R⁴ is phenyl or phenyl substituted by one to five substituents selected from halogen, C₁-C₄alkyl, C₁-C₄haloalkyl: or C₁-C₃alkoxy which may be the same or different. Examples of such groups for R⁴ are 4-bromo-phenyl, 4-chloro-phenyl, 4-cyano-phenyl, 3,4-dichloro-phenyl, 4-fluoro-phenyl, 4-methylthio-phenyl, 4-nitro-phenyl, phenyl, and 4-trifluoromethyl-phenyl.

Most preferably R⁴ is phenyl or phenyl substituted by one substituent selected from halogen, C₁-C₄alkyl, C₁-C₄haloalkyl. Examples of such preferred groups for R⁴ are 4-chloro-phenyl, 4-fluoro-phenyl and 4-trifluoromethyl-phenyl.

Preferably Y¹ is cyano, chloro, methyl, ethyl, or trifluoromethyl

More preferably Y¹ is cyano, chloro, methyl or trifluoromethyl.

Even more preferably Y¹ is methyl or ethyl.

Most preferably Y¹ is methyl.

Preferably Y² is hydrogen, fluoro, chloro or methyl.

Most preferably Y² is hydrogen.

Preferably Y³ is hydrogen, fluoro, chloro or methyl.

Most preferably Y³ is hydrogen.

Preferably Y⁴ is cyano, chloro, methyl, ethyl or trifluoromethyl.

More preferably Y⁴ is cyano, chloro, methyl or trifluoromethyl.

Even more preferably Y⁴ is methyl or ethyl.

Most preferably Y⁴ is methyl.

One preferred embodiment are compounds of formula (Ia) wherein A¹, A², A³, A⁴ are CH.

Another preferred embodiment are compounds of formula (Ib) wherein A¹ is C-F, and A², A³, and A⁴ are CH.

A further preferred embodiment are compounds of formula (Ic) wherein A³ is C-F, and A¹, A², and A⁴ are CH.

Yet another preferred embodiment are compounds of formula (Id) wherein A⁴ is C-F, and A¹, A², and A³ are CH.

In one embodiment of the invention Y¹ and Y⁴ are independently of each other cyano, halogen, C₁-C₄alkyl or C₁-C₄haloalkyl,

In one embodiment of the invention each X is independently hydrogen, halogen, C₁-C₄alkyl or trifluoromethyl.

Certain intermediates are novel and as such form a further aspect of the invention. One group of novel intermediates are compounds of formula (II) 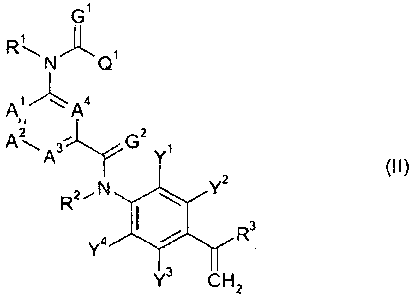 wherein A¹, A², A³, A⁴, R¹, R², R³, G¹, G², Q¹, Y¹, Y², Y³ and Y⁴ are as defined in relation to formula I; or salts or N-oxides thereof. The preferences for A¹, A², A³, A⁴, R¹, R², R³, G¹, G², Q¹, Y¹, Y², Y³ and Y⁴ are the same as the preferences set out for the corresponding substituents of the compounds of the formula (I).

A further group of novel intermediates are compounds of formula (XII) 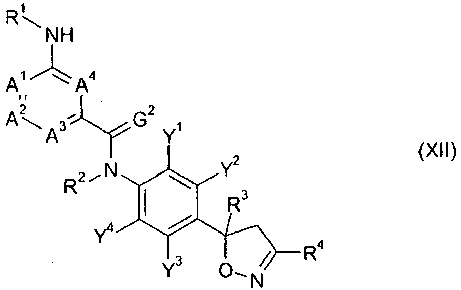 wherein A¹, A², A³, A⁴, R¹, R², R³, R⁴, G², Y¹, Y², Y³ and Y⁴ are as defined in relation to formula I; or salts or N-oxides thereof. The preferences for A¹, A², A³, A⁴, R¹, R², R³, R⁴, G², Y¹, Y², Y³ and Y⁴ are the same as the preferences set out for the corresponding substituents of the compounds of the formula (I).

Another group of novel intermediates are compounds of formula (XIII) 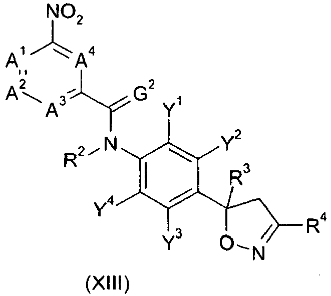 wherein A¹, A², A³, A⁴, R², R³, R⁴, G², Y¹, Y², Y³ and Y⁴ are as defined in relation to formula I; or salts or N-oxides thereof. The preferences for A¹, A², A³, A⁴, R², R³, R⁴, G², Y¹, Y², Y³ and Y⁴ are the same as the preferences set out for the corresponding substituents of the compounds of the formula (I).

A further group of novel intermediates are compounds of formula (XIV) 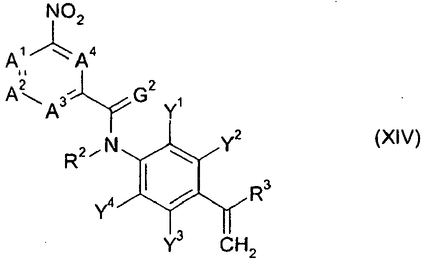 wherein A¹, A², A³, A⁴, R², R³, G², Y¹, Y², Y³ and Y⁴ are as defined in relation to formula I; or salts or N-oxides thereof. The preferences for A¹, A², A³, A⁴, R², R³, G², Y¹, Y², Y³ and Y⁴ are the same as the preferences set out for the corresponding substituents of the compounds of the formula (I).

The compounds in Tables 1 to 22 below illustrate the compounds of the invention.

**Table 1:**

| | |
|---|---|
| Table 1 provides 26 compounds of formula (Ia) wherein R⁴ is 4-bromo-phenyl and Q¹ has the values listed in the table below. | |
| 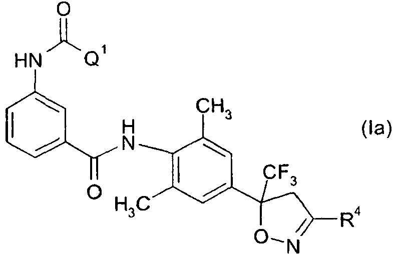 | |

| Compound numbers | Q¹ |
|---|---|
| 1.01 | 5-bromo-furan-2-yl |
| 1.02 | 2-bromo-phenyl |
| 1.03 | 5-bromo-pyrid-3-yl |
| 1.04 | 2-chloro-5-nitro-phenyl |
| 1.05 | 2-chloro-phenyl |
| 1.06 | 3-chloro-phenyl |
| 1.07 | 2-chloro-pyrid-3-yl |
| 1.08 | 2-chloro-pyrid-4-yl |
| 1.09 | 6-chloro-pyrid-3-yl |
| 1.10 | 5-chloro-thiophen-2-yl |
| 1.11 | 3-chloro-5-trifluoromethyl-pyrid-2-yl |
| 1.12 | 4-cyano-phenyl |
| 1.13 | 2,5-dichloro-phenyl |
| 1.14 | 2,3-difluoro-phenyl |
| 1.15 | 1,3-dimethyl-1H-pyrazol-5-yl |
| 1.16 | 4-fluoro-phenyl |
| 1.17 | 2-fluoro-pyrid-3-yl |
| 1.18 | 2-fluoro-3-trifluoromethyl-phenyl |
| 1.19 | 2-methyl-phenyl |
| 1.20 | 3-methyl-pyrid-2-yl |
| 1.21 | 2-methylthio-pyrid-3-yl |
| 1.22 | 4-nitro-phenyl |
| 1.23 | phenyl |
| 1.24 | 1,2,3-thiadiazol-4-yl |
| 1.25 | thiophen-2-yl |
| 1.26 | 4-trifluoromethyl-phenyl |

### Table 2:

Table 2 provides 26 compounds of formula (Ia) wherein R⁴ is 4-chloro-phenyl and Q¹ has the values listed in Table 1.

### Table 3:

Table 3 provides 26 compounds of formula (Ia) wherein R⁴ is 4-cyano-phenyl and Q¹ has the values listed in Table 1.

### Table 4:

Table 4 provides 26 compounds of formula (Ia) wherein R⁴ is 3,4-dichloro-phenyl and Q¹ has the values listed in Table 1.

### Table 5:

Table 5 provides 26 compounds of formula (Ia) wherein R⁴ is 4-difluoromethoxy-phenyl and Q¹ has the values listed in Table 1.

### Table 6:

Table 6 provides 26 compounds of formula (Ia) wherein R⁴ is 4-fluoro-phenyl and Q¹ has the values listed in Table 1.

### Table 7: (Reference)

Table 7 provides 26 compounds of formula (Ia) wherein R⁴ is 4-methylsulfonyloxy-phenyl and Q¹ has the values listed in Table 1.

### Table 8: (Reference)

Table 8 provides 26 compounds of formula (Ia) wherein R⁴ is 4-methylsulfonyl-phenyl and Q¹ has the values listed in Table 1.

### Table 9:

Table 9 provides 26 compounds of formula (Ia) wherein R⁴ is 4-methylthio-phenyl and Q¹ has the values listed in Table 1.

### Table 10:

Table 10 provides 26 compounds of formula (Ia) wherein R⁴ is 4-nitro-phenyl and Q¹ has the values listed in Table 1.

### Table 11:

Table 11 provides 26 compounds of formula (Ia) wherein R⁴ is phenyl and Q¹ has the values listed in Table 1.

### Table 12: (Reference)

Table 12 provides 26 compounds of formula (Ia) wherein R⁴ is 4-trifluoromethoxy-phenyl and Q¹ has the values listed in Table 1.

### Table 13:

Table 13 provides 26 compounds of formula (Ia) wherein R⁴ is 4-trifluoromethyl-phenyl and Q¹ has the values listed in Table 1.

### Table 14:

Table 14 provides 26 compounds of formula (Ib) wherein R⁴ is 4-chloro-phenyl and Q¹ has the values listed in Table 1.

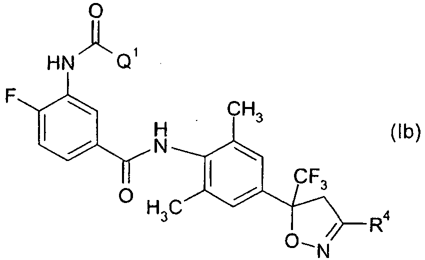

### Table 15:

Table 15 provides 26 compounds of formula (Ib) wherein R⁴ is 4-fluoro-phenyl and Q¹ has the values listed in Table 1.

### Table 16:

Table 16 provides 26 compounds of formula (Ib) wherein R⁴ is 4-trifluoromethyl-phenyl and Q¹ has the values listed in Table 1.

### Table 17:

Table 17 provides 26 compounds of formula (Ic) wherein R⁴ is 4-chloro-phenyl and Q¹ has the values listed in Table 1.

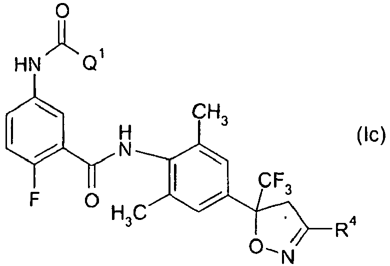

### Table 18:

Table 18 provides 26 compounds of formula (Ic) wherein R⁴ is 4-fluoro-phenyl and Q¹ has the values listed in Table 1.

### Table 19:

Table 19 provides 26 compounds of formula (Ic) wherein R⁴ is 4-trifluoromethyl-phenyl and Q¹ has the values listed in Table 1.

### Table 20:

Table 20 provides 26 compounds of formula (Id) wherein R⁴ is 4-chloro-phenyl and Q¹ has the values listed in Table 1.

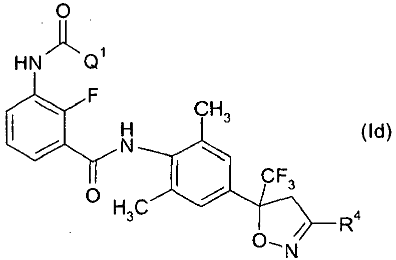

### Table 21:

Table 21 provides 26 compounds of formula (Id) wherein R⁴ is 4-fluoro-phenyl and Q¹ has the values listed in Table 1.

### Table 22:

Table 22 provides 26 compounds of formula (Id) wherein R⁴ is 4-trifluoromethyl-phenyl and Q¹ has the values listed in Table 1.

The compounds of the invention may be made by a variety of methods.
1) Compounds of formula (I), wherein G¹ and G² are oxygen, may be made by treatment of a compound of formula (II), wherein G¹ and G² are oxygen, with a hydroxyl-oxime of formula (III) in two steps. 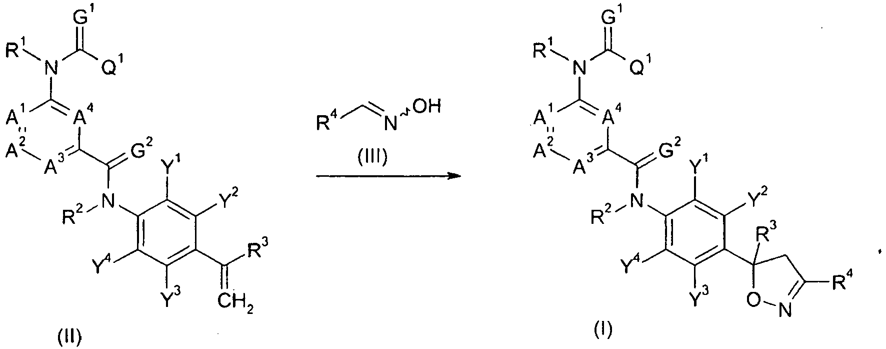 First the hydroxyl-oxime of formula (III) is reacted with a halogenating agent, such as N-chlorosuccinimide, to form a vinyl halide. Then the vinyl halide is reacted with a compound of formula (II) in the presence of a base, such as triethylamine. Such procedures are known, for example, from Indian Journal of Chemistry, Section B (1993), 32B(4), 471-474; and Current Organic Chemistry (2005), 9(10), 925-958. Hydroxyl-oximes of formula (III) are commercially available or may be made by methods known to a person skilled in the art.
2) Compounds of formula (II), wherein G¹ and G² are oxygen, may be made by treatment of a compound of formula (IV), wherein G¹ is oxygen and R is OH, C₁-C₆alkoxy or Cl, F or Br with an amine of formula (V). 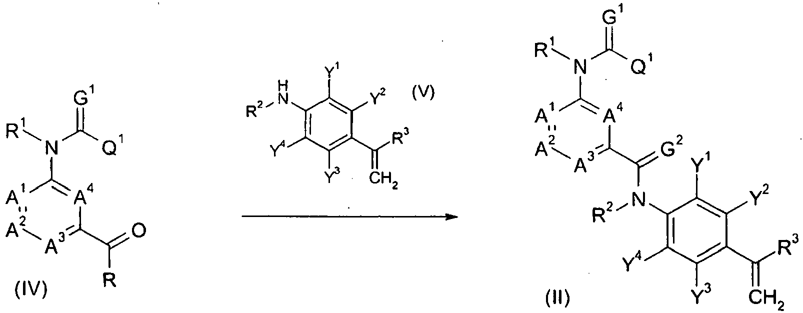 When R is OH such reactions are usually carried out in the presence of a coupling reagent, such as DCC (N,N'-dicyclohexylcarbodiimide), EDC (1-ethyl-3-[3-dimethylaminopropyl]carbodiimide hydrochloride) or BOP-Cl (bis(2-oxo-3-oxazolidinyl)phosphonic chloride), in the presence of a base, such as pyridine, triethylamine, 4-(dimethylamino)-pyridine or diisopropylethylamine, and optionally in the presence of a nucleophilic catalyst, such as hydroxybenzotriazole. When R is Cl, such reactions are usually carried out under basic conditions (for example in the presence of pyridine, triethylamine, 4-(dimethylamino)-pyridine or diisopropylethylamine), again optionally in the presence of a nucleophilic catalyst. Alternatively, it is possible to conduct the reaction in a biphasic system comprising an organic solvent, preferably ethyl acetate, and an aqueous solvent, preferably a solution of sodium bicarbonate. When R is C₁-C₆alkoxy it is sometimes possible to convert the ester directly to the amide by heating the ester and amine together in a thermal process.
3) Acid halides of formula (IV), wherein G¹ is oxygen and R is Cl, F or Br, may be made from a carboxylic acid of formula (IV), wherein G¹ is oxygen and R is OH, under standard conditions, such as treatment with thionyl chloride or oxalyl chloride.
4) Carboxylic acids of formula (IV), wherein G¹ is oxygen and R is OH, may be formed from an ester of formula (IV), wherein G¹ is oxygen and R is C₁-C₆alkoxy. It is known to a person skilled in the art that there are many methods for the hydrolysis of such esters depending on the nature of the alkoxy group. One widely used method to achieve such a transformation is the treatment of the ester with an alkali hydroxide, such as sodium hydroxide, in a solvent, such as ethanol.
5) Amines of formula (V) may be made from an amine of formula (VI) wherein X^{A} is a leaving group such as a halogen, preferably bromine, via a two step process. First the amine of formula (VI) is reacted with a boron reagent, such as bis(pinacolato)diboron, in the presence of a catalyst/ligand system, often a palladium(II) complex, in the presence of a base under an inert atmosphere. Such procedures are known, for example, from Palladium(0)-Catalyzed Cross-Coupling Reaction of Alkoxydiboron with Haloarenes: A Direct Procedure for Arylboronic Esters; Ishiyama, Tatsuo; Murata, Miki; Miyaura, Norio; and Journal of Organic Chemistry (1995), 60(23), 7508-10. 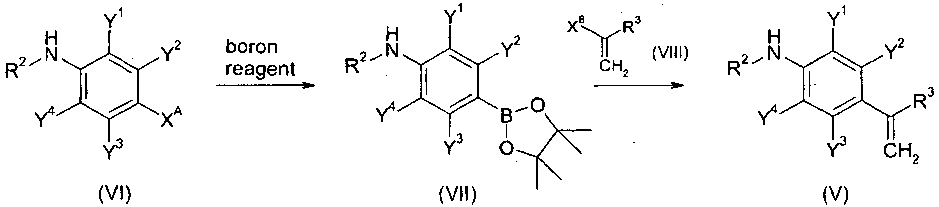 Then the boronic ester of formula (VII) is then reacted with a vinyl halide of formula (VIII) in the presence of a suitable catalyst/ligand system, often a palladium(II) complex, in the presence of a base under an inert atmosphere. Such procedures are known, for example, from WO 02/08221. Vinyl halides of formula (VIII) are commercially available or may be made by methods known to a person skilled in the art.
6) Compounds of formula (IV), wherein G¹ is oxygen and R is C₁-C₆alkoxy, may be made from of a compound of formula (IX), wherein R is C₁-C₆alkoxy, by acylation with a carboxylic acid of formula Q¹-COOH or an acid halide of formula Q¹-COHal, wherein Hal is Cl, F or Br, under standard conditions as described in 2). 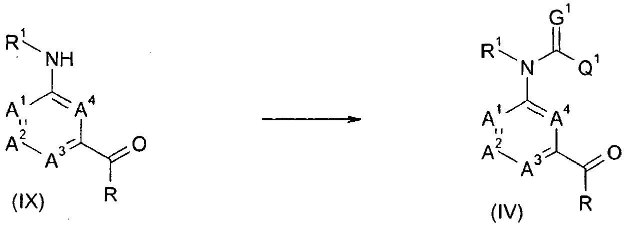
7) For compounds of formula (IV), the esters (wherein R is C₁-C₆alkoxy) may be hydrolysed to the acids (wherein R is OH) by treatment with an alkali hydroxide, such as sodium hydroxide, in a solvent, such as ethanol as described in 4). The acids (wherein R is OH) may be converted to the acid chlorides (wherein R is Cl) by treatment with thionyl chloride or oxalyl chloride as described in 3).
8) Compounds of formula (IX), wherein R is C₁-C₆alkoxy, may be made from a compound of formula (X) by sequential treatment with an alcohol R-OH under acidic conditions and then formation of the N-R¹ bond. It is known to a person skilled in the art that there are many reported methods for the formation of this bond depending on the nature of the substituent R¹. 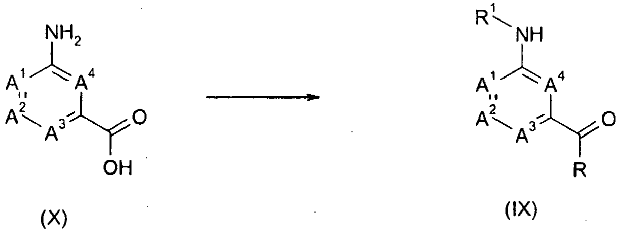 For example, reductive amination may be achieved by treatment of the amine with an aldehyde or ketone and a reducing agent such as sodium cyanoborohydride. Alternatively, alkylation may be achieved by treating the amine with an alkylating agent such as an alkyl halide, optionally in the presence of a base. Alternatively, arylation may be achieved by treatment of the amine with an aryl halide or sulfonate in the presence of a suitable catalyst/ligand system, often a palladium(0) complex. Compounds of formula (X) are either known compounds or may be made by methods known to a person skilled in the art.
9) Alternatively, compounds of formula (IX), wherein R is C₁-C₆alkoxy, may be made from a compound of formula (XI), wherein R is C₁-C₆alkoxy and LG is a leaving group, such as fluoro, chloro or sulfonate, via nucleophilic displacement of the leaving group by an amine of formula R¹-NH₂. 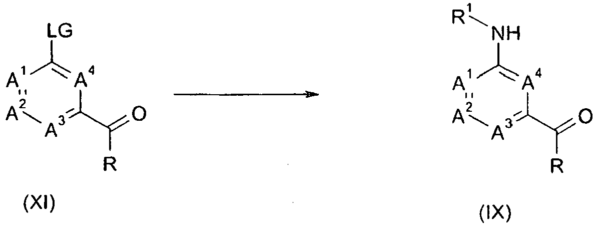 Compounds of formula (XI) and amines of formula R¹-NH₂ are either known compounds or may be made by methods known to a person skilled in the art.
10) Alternatively, compounds of formula (I), wherein G¹ and G² are oxygen, may be made from a compound of formula (XII), wherein G² is oxygen, 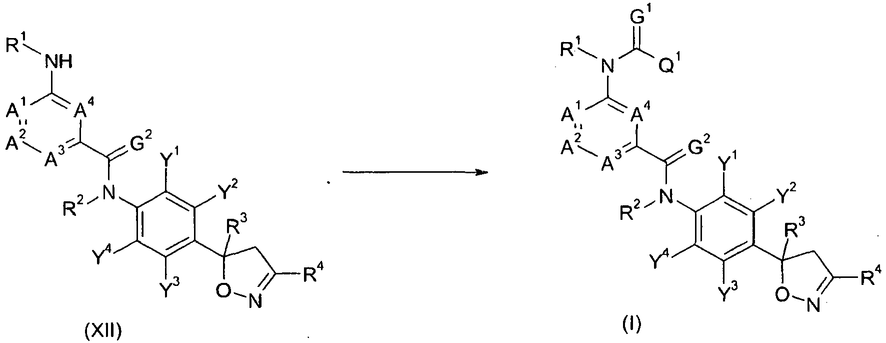 by acylation with a carboxylic acid of formula Q¹-COOH or an acid halide of formula Q¹-COHal, wherein Hal is Cl, F or Br, under standard conditions as described in 2).
11) Compounds of formula (XII), wherein G² is oxygen and R¹ is hydrogen, may be made by the reduction of a nitro compound of formula (XIII), wherein G² is oxygen. 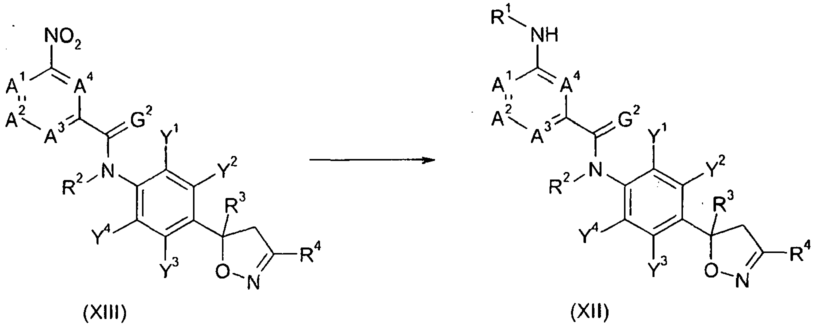 There are numerous methods for achieving such a transformation reported in the literature such as treatment with tin chloride under acidic conditions, or hydrogenation catalysed by a noble metal such as palladium on carbon.
12) Compounds of formula (XIII), wherein G² is oxygen, may be derived from a compound of formula (XIV), wherein G² is oxygen, by treatment with a hydroxyl-oxime of formula (III) in two steps as described in 1). 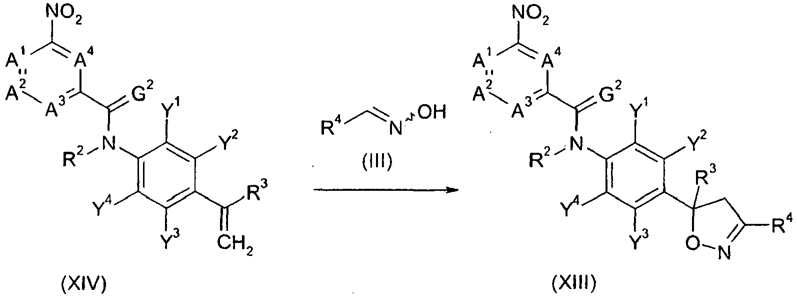
13) Compounds of formula (XIV), wherein G² is oxygen, may be made by treatment of a compound of formula (XV), wherein R is OH, C₁-C₆alkoxy or Cl, F or Br with an amine of formula (V) under standard conditions as described in 2). 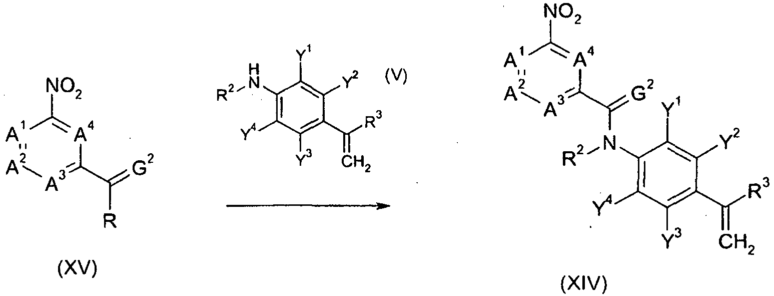 Compounds of formula (XV) are either commercially available or can be made my methods known to a person skilled in the art.
14) Compounds of formula (1), wherein G¹ and G² are sulfur, may be made from a compound of formula (I), wherein G¹ and G² are oxygen, by treatment with a thio-transfer reagent, such as Lawesson's reagent or phosphorus pentasulfide.
15) Compounds of formula (I), wherein G¹ is sulfur and G² is oxygen, may be made from a compound of formula (IV), wherein G¹ is oxygen and R is OH or C₁-C₆alkoxy, by treatment with a thio-transfer reagent, such as Lawessen's reagent or phosphorus pentasulfide, prior to coupling with the amine of formula (V).
16) Compounds of formula (I), wherein G¹ is oxygen and G² is sulfur, may be made from a compound of formula (XII), wherein G² is oxygen, by treatment with a thio-transfer reagent, such as Lawessen's reagent or phosphorus pentasulfide, prior to acylation with a carboxylic acid of formula Q¹-COOH or an acid halide of formula Q¹-COHal, wherein Hal is Cl, F or Br.

The compounds of formula (I) can be used to combat and control infestations of insect pests such as Lepidoptera, Diptera, Hemiptera, Thysanoptera, Orthoptera, Dictyoptera, Coleoptera, Siphonaptera, Hymenoptera and Isoptera and also other invertebrate pests, for example, acarine, nematode and mollusc pests. Insects, acarines, nematodes and molluscs are hereinafter collectively referred to as pests. The pests which may be combated and controlled by the use of the invention compounds include those pests associated with agriculture (which term includes the growing of crops for food and fibre products), horticulture and forestry and the storage of products of vegetable origin (such as fruit, grain and timber); those pests associated with the damage of man-made structures and the transmission of diseases of man and animals; and also nuisance pests (such as flies).

Examples of pest species which may be controlled by the compounds of formula (I) include: *Myzus persicae* (aphid), *Aphis gossypii* (aphid), *Aphis fabae* (aphid), *Lygus* spp. (capsids), *Dysdercus* spp. (capsids), *Nilaparvata lugens* (planthopper), *Nephotettixc incticeps* (leafhopper), *Nezara* spp. (stinkbugs), *Euschistus* spp. (stinkbugs), *Leptocorisa* spp. (stinkbugs), *Frankliniella occidentalis* (thrip), Thrips spp. (thrips), *Leptinotarsa decemlineata* (Colorado potato beetle), *Anthonomus grandis* (boll weevil), *Aonidiella* spp. (scale insects), *Trialeurodes* spp. (white flies), *Bemisia tabaci* (white fly), *Ostrinia nubilalis* (European corn borer), *Spodoptera littoralis* (cotton leafworm), *Heliothis virescens* (tobacco budworm), *Helicoverpa armigera* (cotton bollworm), *Helicoverpa zea* (cotton bollworm), *Sylepta derogata* (cotton leaf roller), *Pieris brassicae* (white butterfly), *Plutella xylostella* (diamond back moth), *Agrotis* spp. (cutworms), *Chilo suppressalis* (rice stem borer), *Locusta. migratoria* (locust), *Chortiocetes terminifera* (locust), *Diabrotica* spp. (rootworms), *Panonychus ulmi* (European red mite), *Panonychus citri* (citrus red mite), *Tetranychus urticae* (two-spotted spider mite), *Tetranychus cinnabarinus* (carmine spider mite), *Phyllocoptruta oleivora* (citrus rust mite), *Polyphagotarsonemus latus* (broad mite), *Brevipalpus* spp. (flat mites), *Boophilus microplus* (cattle tick), *Dermacentor variabilis* (American dog tick), *Ctenocephalides felis* (cat flea), *Liriomyza* spp. (leafminer), *Musca domestica* (housefly), *Aedes aegypti* (mosquito), *Anopheles* spp. (mosquitoes), *Culex* spp. (mosquitoes), *Lucillia* spp. (blowflies), *Blattella germanica* (cockroach), *Periplaneta americana* (cockroach), *Blatta orientalis* (cockroach), termites of the Mastotermitidae (for example *Mastotermes* spp.), the Kalotermitidae (for example *Neotermes* spp.), the Rhinotermitidae (for example *Coptotermes formosanus, Reticulitermes flavipes, R. speratu, R. virginicus, R. hesperus,* and *R. santonensis)* and the Termitidae (for example *Globitermes sulfureus), Solenopsis geminata* (fire ant), *Monomorium pharaonis* (pharaoh's ant), *Damalinia* spp. and *Linognathus* spp. (biting and sucking lice), *Meloidogyne* spp. (root knot nematodes), *Globodera* spp. and *Heterodera* spp. (cyst nematodes), *Pratylenchus* spp. (lesion nematodes), *Rhodopholus* spp. (banana burrowing nematodes), *Tylenchulus* spp.(citrus nematodes), *Haemonchus contortus* (barber pole worm), *Caenorhabditis elegans*_(vinegar eelworm), *Trichostrongylus* spp. (gastro intestinal nematodes) and *Deroceras reticulatum* (slug).

The invention therefore provides a method of combating and controlling insects, acarines, nematodes or molluscs which comprises applying an insecticidally, acaricidally, nematicidally or molluscicidally effective amount of a compound of formula (I), or a composition containing a compound of formula (I), to a pest, a locus of pest, preferably a plant, or to a plant susceptible to attack by a pest, The compounds of formula (I) are preferably used against insects, acarines or nematodes.

The term "plant" as used herein includes seedlings, bushes and trees.

Crops are to be understood as also including those crops which have been rendered tolerant to herbicides or classes of herbicides (e.g. ALS-, GS-, EPSPS-, PPO- and HPPD-inhibitors) by conventional methods of breeding or by genetic engineering. An example of a crop that has been rendered tolerant to imidazolinones, e.g. imazamox, by conventional methods of breeding is Clearfield® summer rape (canola). Examples of crops that have been rendered tolerant to herbicides by genetic engineering methods include e.g. glyphosate- and glufosinate-resistant maize varieties commercially available under the trade names RoundupReady® and LibertyLink®.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK® (Syngenta Seeds). Examples of transgenic plants comprising one or more genes that code for an insecticidal resistance and express one or more toxins are Knockout® (maize), Yield Gard® (maize), NuCOTIN33B® (cotton), Bollgard® (cotton), NewLeaf® (potatoes), NatureGard® and Protexcta®.

Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood as being those which are obtained by conventional methods of breeding or genetic engineering and contain so-called output traits (e.g. improved storage stability, higher nutritional value and improved flavour).

In order to apply a compound of formula (I) as an insecticide, acaricide, nematicide or molluscicide to a pest, a locus of pest, or to a plant susceptible to attack by a pest, a compound of formula (I) is usually formulated into a composition which includes, in addition to the compound of formula (I), a suitable inert diluent or carrier and, optionally, a surface active agent (SFA). SFAs are chemicals which are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (I). The composition is generally used for the control of pests such that a compound of formula (I) is applied at a rate of from 0.1 g to 10kg per hectare, preferably from 1g to 6kg per hectare, more preferably from 1g to 1kg per hectare.

When used in a seed dressing, a compound of formula (I) is used at a rate of 0.0001 g to 10g (for example 0.001g or 0.05g), preferably 0.005g to 10g, more preferably 0.005g to 4g, per kilogram of seed.

In another aspect the present invention provides an insecticidal, acaricidal, nematicidal or molluscicidal composition comprising an insecticidally, acaricidally, nematicidally or molluscicidally effective amount of a compound of formula (I) and a suitable carrier or diluent therefor. The composition is preferably an insecticidal, acaricidal, nematicidal or molluscicidal composition.

The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (I).

Dustable powders (DP) may be prepared by mixing a compound of formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulfur, lime, flours, talc and other organic and inorganic solid carriers) and mechanically grinding the mixture to a fine powder.

Soluble powders (SP) may be prepared by mixing a compound of formula (I) with one or more water-soluble inorganic salts (such as sodium bicarbonate, sodium carbonate or magnesium sulfate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (I) and one or more powdered solid diluents or carriers, or from pre-formed blank granules by absorbing a compound of formula (I) (or a solution thereof, in a suitable agent) in a porous granular material (such as pumice, attapulgite clays, fuller's earth, kieselguhr, diatomaceous earths or ground corn cobs) or by adsorbing a compound of formula (I) (or a solution thereof, in a suitable agent) on to a hard core material (such as sands, silicates, mineral carbonates, sulfates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (I) either as a liquid (if it is not a liquid at room temperature, it may be melted at a reasonable temperature, typically below 70°C) or in solution (by dissolving it in an appropriate solvent) and then emulsifiying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of formula (I) is present initially in either the water or the solvent/SFA blend. Suitable solvents for use in MEs include those hereinbefore described for use in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (I). SCs may be prepared by.ball or bead milling the solid compound of formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (I) may be dry milled and added to water, containing agents hereinbefore described, to produce the desired end product.

Aerosol formulations comprise a compound of formula (I) and a suitable propellant (for example n-butane). A compound of formula (I) may also be dissolved or dispersed in a suitable medium (for example water or a water miscible liquid, such as n-propanol) to provide compositions for use in non-pressurised, hand-actuated spray pumps.

A compound of formula (I) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (I) and, optionally, a carrier or diluent therefor. The polymeric shell may be produced by either an interfacial polycondensation reaction or by a coacervation procedure. The compositions may provide for controlled release of the compound of formula (I) and they may be used for seed treatment. A compound of formula (I) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (I)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (I)).

A compound of formula (I) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be surface SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulfuric acid (for example sodium lauryl sulfate), salts of sulfonated aromatic compounds (for example sodium dodecylbenzenesulfonate, calcium dodecylbenzenesulfonate, butylnaphthalene sulfonate and mixtures of sodium di-isopropyl- and tri-isopropyl-naphthalene sulfonates), ether sulfates, alcohol ether sulfates (for example sodium laureth-3-sulfate), ether carboxylates (for example sodium laureth-3-carboxylate), phosphate esters (products from the reaction between one or more fatty alcohols and phosphoric acid (predominately mono-esters) or phosphorus pentoxide (predominately diesters), for example the reaction between lauryl alcohol and tetraphosphoric acid; additionally these products may be ethoxylated), sulfosuccinamates, paraffin or olefine sulfonates, taurates and lignosulfonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpytrolidone or sodium carboxymethylcellulose) and swelling clays (such as bentonite or attapulgite).

A compound of formula (I) may be applied by any of the known means of applying pesticidal compounds. For example, it may be applied, formulated or unformulated, to the pests or to a locus of the pests (such as a habitat of the pests, or a growing plant liable to infestation by the pests) or to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula (I) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs, SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (I) (for example 0.0001 to 10%, by weight) depending upon the purpose for which they are to be used.

A compound of formula (I) may be used in mixtures with fertilisers (for example nitrogen-, potassium- or phosphorus-containing fertilisers). Suitable formulation types include granules of fertiliser. The mixtures preferably contain up to 25% by weight of the compound of formula (I).

The invention therefore also provides a fertiliser composition comprising a fertiliser and a compound of formula (I).

The compositions of this invention may contain other compounds having biological activity, for example micronutrients or compounds having fungicidal activity or which possess plant growth regulating, herbicidal, insecticidal, nematicidal or acaricidal activity.

The compound of formula (I) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (I); or help to overcome or prevent the development of resistance to individual components. The particular additional active ingredient will depend upon the intended utility of the composition. Examples of suitable pesticides include the following:
a) Pyrethroids, such as permethrin, cypermethrin, fenvalerate, esfenvalerate, deltamethrin, cyhalothrin (in particular lambda-cyhalothrin), bifenthrin, fenpropathrin, cyfluthrin, tefluthrin, fish safe pyrethroids (for example ethofenprox), natural pyrethrin, tetramethrin, s-bioallethrin, fenfluthrin, prallethrin or 5-benzyl-3-furylmethyl-(E)-(1R,3S)-2,2-dimethyl-3-(2-oxothiolan-3-ylidenemethyl)cyclopropane carboxylate;
b) Organophosphates, such as, profenofos, sulprofos, acephate, methyl parathion, azinphos-methyl, demeton-s-methyl, heptenophos, thiometon, fenamiphos, monocrotophos, profenofos, triazophos, methamidophos, dimethoate, phosphamidon, malathion, chlorpyrifos, phosalone, terbufos, fensulfothion, fonofos, phorate, phoxim, pirimiphos-methyl, pirimiphos-ethyl, fenitrothion, fosthiazate or diazinon;
c) Carbamates (including aryl carbamates), such as pirimicarb, triazamate, cloethocarb, carbofuran, furathiocarb, ethiofencarb, aldicarb, thiofurox, carbosulfan, bendiocarb, fenobucarb, propoxur, methomyl or oxamyl;
d) Benzoyl ureas, such as diflubenzuron, triflumuron, hexaflumuron, flufenoxuron or chlorfluazuron;
e) Organic tin compounds, such as cyhexatin, fenbutatin oxide or azocyclotin;
f) Pyrazoles, such as tebufenpyrad and fenpyroximate;
g) Macrolides, such as avermectins or milbemycins, for example abamectin, emamectin benzoate, ivermectin, milbemycin, spinosad or azadirachtin;
h) Hormones or pheromones;
i) Organochlorine compounds such as endosulfan, benzene hexachloride, DDT, chlordane or dieldrin;
j) Amidines, such as chlordimeform or amitraz;
k) Fumigant agents, such as chloropicrin, dichloropropane, methyl bromide or metam;
l) Neonicotinoid compounds such as imidacloprid, thiacloprid, acetamiprid, nitenpyram, dinotefuran or thiamethoxam;
m) Diacylhydrazines, such as tebufenozide, chromafenozide or methoxyfenozide;
n) Diphenyl ethers, such as diofenolan or pyriproxifen;
o) Indoxacarb;
p) Chlorfenapyr;
q) Pymetrozine;
r) Spirotetramat, spirodiclofen or spiromesifen; or
s) Flubendiamid or rynaxypyr

In addition to the major chemical classes of pesticide listed above, other pesticides having particular targets may be employed in the composition, if appropriate for the intended utility of the composition. For instance, selective insecticides for particular crops, for example stemborer specific insecticides (such as cartap) or hopper specific insecticides (such as buprofezin) for use in rice may be employed. Alternatively insecticides or acaricides specific for particular insect species/stages may also be included in the compositions (for example acaricidal ovo-larvicides, such as clofentezine, flubenzimine, hexythiazox or tetradifon; acaricidal motilicides, such as dicofol or propargite; acaricides, such as bromopropylate or chlorobenzilate; or growth regulators, such as hydramethylnon, cyromazine, methoprene, chlorfluazuron or diflubenzuron).

Examples of fungicidal compounds which may be included in the composition of the invention are (E)-*N*-methyl-2-[2-(2,5-dimethylphenoxymethyl)phenyl]-2-methoxy-iminoacetamide (SSF-129), 4-bromo-2-cyano-*N*,*N*-dimethyl-6-trifluoromethylbenzimidazole-1-sulfonamide, α-[*N*-(3-chloro-2,6-xylyl)-2-methoxyacetamido]-γ-butyrolactone, 4-chloro-2-cyano-*N*,*N*-dimethyl-5-p-tolylimidazole-1-sulfonamide (IKF-916, cyamidazosulfamid), 3-5-dichloro-*N*-(3-chloro-1-ethyl-1-methyl-2-oxopropyl)-4-methylbenzamide (RH-7281, zoxamide), *N*-allyl-4,5,-dimethyl-2-trimethylsilylthiophene-3-carboxamide (MON65500), *N-*(1-cyano-1,2-dimethylpropyl)-2-(2,4-dichlorophenoxy)propionamide (AC382042), *N*-(2-methoxy-5-pyridyl)-cyclopropane carboxamide, acibenzolar (CGA245704), alanycarb, aldimorph, anilazine, azaconazole, azoxystrobin, benalaxyl, benomyl, biloxazol, bitertanol, blasticidin S, bromuconazole, bupirimate, captafol, captan, carbendazim, carbendazim chlorhydrate, carboxin, carpropamid, carvone, CGA41396, CGA41397, chinomethionate, chlorothalonil, chlorozolinate, clozylacon, copper containing compounds such as copper oxychloride, copper oxyquinolate, copper sulfate, copper tallate and Bordeaux mixture, cymoxanil, cyproconazole, cyprodinil, debacarb, di-2-pyridyl disulfide 1,1'-dioxide, dichlofluanid, diclomezine, dicloran, diethofencarb, difenoconazole, difenzoquat, diflumetorim, O,O-di-iso-propyl-S-benzyl thiophosphate, dimefluazole, dimetconazole, dimethomorph, dimethirimol, diniconazole, dinocap, dithianon, dodecyl dimethyl ammonium chloride, dodemorph, dodine, doguadine, edifenphos, epoxiconazole, ethirimol, ethyl(Z)-*N*-benzyl-*N*([methyl(methyl-thioethylideneaminooxycarbonyl)]thio)-β -alaninate, etridiazole, famoxadone, fenamidone (RPA407213), fenarimol, fenbuconazole, fenfuram, fenhexamid (KBR2738), fenpiclonil, fenpropidin, fenpropimorph, fentin acetate, fentin hydroxide, ferbam, ferimzone, fluazinam, fludioxonil, flumetover, fluoroimide, fluquinconazole, flusilazole, flutolanil, flutriafol, folpet, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb (SZX0722), isopropanyl butyl carbamate, isoprothiolane, kasugamycin, kresoxim-methyl, LY186054, LY211795, LY248908, mancozeb, maneb, mefenoxam, mepanipyrim, mepronil, metalaxyl, metconazole, metiram, metiram-zinc, metominostrobin, myclobutanil, neoasozin, nickel dimethyldithiocarbamate, nitrothal-isopropyl, nuarimol, ofurace, organomercury compounds, oxadixyl, oxasulfuron, oxolinic acid, oxpoconazole, oxycarboxin, pefurazoate, penconazole, pencycuron, phenazin oxide, phosetyl-Al, phosphorus acids, phthalide, picoxystrobin (ZA1963), polyoxin D, polyram, probenazole, prochloraz, procymidone, propamocarb, propiconazole, propineb, propionic acid, pyrazophos, pyrifenox, pyrimethanil, pyroquilon, pyroxyfur, pyrrolnitrin, quaternary ammonium compounds, quinomethionate, quinoxyfen, quintozene, sipconazole (F-155), sodium pentachlorophenate, spiroxamine, streptomycin, sulfur, tebuconazole, tecloftalam, tecnazene, tetraconazole, thiabendazole, thifluzamid, 2-(thiocyanomethylthio)benzothiazole, thiophanate-methyl, thiram, timibenconazole, tolclofos-methyl, tolylfluanid, triadimefon, triadimenol, triazbutil, triazoxide, tricyclazole, tridemorph, trifloxystrobin (CGA279202), triforine, triflumizole, triticonazole, validamycin A, vapam, vinclozolin, zineb and ziram.

The compounds of formula (I) may be mixed with soil, peat or other rooting media for the protection of plants against seed-borne, soil-borne or foliar fungal diseases.

Examples of suitable synergists for use in the compositions include piperonyl butoxide, sesamex, safroxan and dodecyl imidazole.

Suitable herbicides and plant-growth regulators for inclusion in the compositions will depend upon the intended target and the effect required.

An example of a rice selective herbicide which may be included is propanil. An example of a plant growth regulator for use in cotton is PIX™.

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The following Examples illustrate, but do not limit, the invention.

### Preparation Examples

### Example I1 : Preparation of 2,6-dimethyl-4-(4,4,5,5-tetramethyl-1,3,2-dioxaborolan-2-yl)-phenylamine

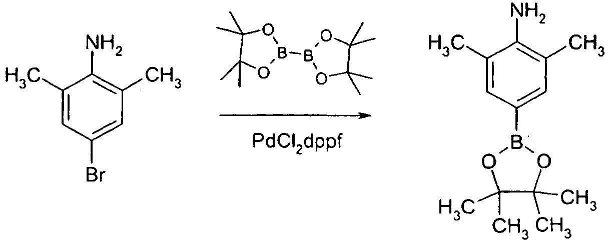

4-Bromo-2,6-dimethyl-phenylamine (2.00 g, 10.0 mmol), bis(pinacolato)diboron (2.79 g, 11.0 mmol), 1,1'-bis(diphenylphosphino)ferrocene palladium(II) dichloride (PdCl₂dppf) (crystallised with dichloromethane 1:1) (0.16 g, 0.2 mmol), 1,1'-bis(diphenylphosphino)ferrocene (0.11 g, 0.2 mmol) and potassium acetate (2.94 g, 30.0 mmol) were dissolved under an argon atmosphere in absolute dioxane (30 ml). The reaction mixture was stirred for 18 hours at 80°C. The reaction mixture was allowed to cooled ambient temperature and filtered through a plug of Celite®. The filtrate was diluted with water (100 ml) and dichloromethane (100 ml) and the phases were separated. The organic phase was washed twice with water, and water phases were extracted twice with dichloromethane. The organic phases were combined, dried over sodium sulfate and concentrated. Purification by chromatography over silica gel (eluent: hexane / ethyl acetate 4:1) gave 2,6-dimethyl-4-(4,4,5,5-tetramethyl-1,3,2-dioxaborolan-2-yl)phenylamine (3.13 g, 87% yield).
¹H-NMR (CDCl₃; 400 MHz): 7.4 (s, 2H), 4.0 (s, 2H), 2.2 (s, 6H), 1.3 (s, 12H).

### Example I2: Preparation of 2,6-dimethyl-4-(1-trifluoromethyl-vinyl)-phenylamine

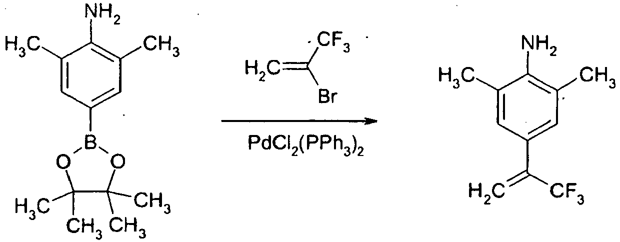

In a microwave vial, 2,6-dimethyl-4-(4,4,5,5-tetramethyl-1,3,2-dioxaborolan-2-yl)phenylamine (200 mg, 0.81 mmol) (Example I1) was dissolved in 1,2-dimethoxyethane (1.2 ml) and tetrahydrofuran (1.2 ml). Then, at 0°C under an argon atmosphere, 2-bromo-3,3,3-trifluoro-propene (167 µl, 1.62 mmol), bis(triphenylphosphine) palladium(II) dichloride (PdCl₂(PPh₃)₂) (18 mg, 0.03 mmol) and triphenylphosphine (32 mg, 0.12 mmol) were added. Finally an aqueous solution of sodium hydroxide (2M) (1.6 ml) was added at 0°C under an argon atmosphere. The vial was sealed and heated in a microwave oven at 130°C for 10 minutes. The reaction mixture was allowed to cool to ambient temperature and concentrated. The residue was suspended in ethyl acetate (30 ml) and filtered through a plug of Celite®. The filtrate was washed twice with water (30 ml). The water phases were extracted with ethyl acetate (30 ml). The organic phases were combined, dried over sodium sulfate and concentrated. Purification by preparative normal phase HPLC gave 2,6-dimethyl-4-(1-trifluoromethyl-vinyl)-phenylamine (140 mg, 80% yield).
¹H-NMR (CDCl₃, 400 MHz): 7.1 (s, 2H), 5.75 (s, 1H), 5.6 (s, 1H), 3.9 (s, 2H), 2.2 (s, 6H).

### Example I3: Preparation of 3-(2-methyl-benzoylamino)-benzoic acid methyl ester

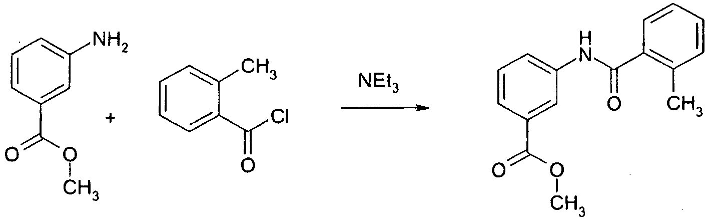

3-Amino-benzoic acid methyl ester (1.47 g, 9.7 mmol) was dissolved in toluene (7.5 ml). Triethylamine (2.03 ml, 29.1 mmol) was added. Then at 0°C, a solution of 2-methylbenzoyl chloride in toluene (7.5 ml) was added slowly. The reaction mixture was stirred at 0°C for 2 hours and then at ambient temperature for 2 hours. The reaction mixture was diluted with ethyl acetate (80 ml) and washed once with hydrochloric acid (0.5M) (70 ml), once with water (70 ml) and once with brine (70 ml). The aqueous phases were extracted with ethyl acetate (70 ml). The organic phases were combined, dried over sodium sulfate and concentrated to give 3-(2-methyl-benzoylamino)-benzoic acid methyl ester (2.48 g, 95% yield).
¹H-NMR (CDCl₃, 400 MHz): 8.1 (s, 1H), 8.05 (d, 1H), 7.8 (dd, 1H), 7.7 (s, 1H), 7.5 (d, 1H), 7.45 (d, 1H), 7.35 (dd, 1H), 7.25 (dd, 2H), 3.9 (s, 3H), 2.5 (s, 3H).

3-(4-Cyano-benzoylamino)-benzoic acid methyl ester, 3-(2,3-difluoro-benzoylamino)-benzoic acid methyl ester and 3-(4-fluoro-benzoylamino)-benzoic acid methyl ester were made using analogous methods.

### Example I4: Preparation of 3-(2-methyl-benzoylamino)-benzoic acid

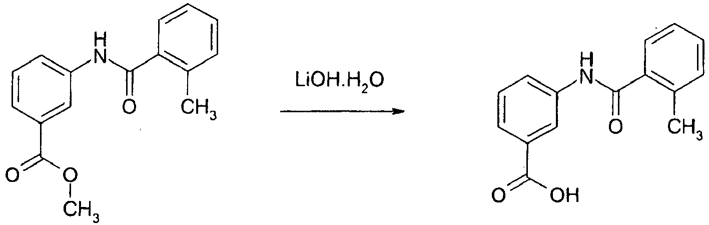

3-(2-Methyl-benzoylamino)-benzoic acid methyl ester (2.476 g, 9.2 mmol) (Example I3) was dissolved in tetrahydrofuran (18 ml). A suspension of lithium hydroxide monohydrate (0.772 g, 18.4 mmol) in water (4 ml) was added. The reaction mixture was stirred at ambient temperature for 18 hours. The reaction mixture was diluted with water (20 ml) and the tetrahydrofuran was removed by evaporation. The aqueous solution was acidified with hydrochloric acid (2M) (20 ml). The precipitate was isolated by filtration and dried under high vacuum, giving 3-(2-methyl-benzoylamino)-benzoic acid (2.037 g, 87% yield).
¹H-NMR (d-DMSO, 400 MHz): 8.4 (s, 1H), 7.9 (d, 1H), 7.6 (d, 1H), 7.4(m, 2H), 7.3(dd, 1H), 7.25 (d, 2H), 2.3 (s, 3H).

3-(4-Cyano-benzoylamino)-benzoic acid, 3-(2,3-difluoro-benzoylamino)-benzoic acid and 3-(4-fluoro-benzoylamino)-benzoic acid were made using analogous methods.

### Example I5: Preparation of 2-methyl-benzoic acid 3-[2,6-dimethyl-4-(1-trifluoromethylvinyl)-phenylcarbamoyl]-phenyl amide

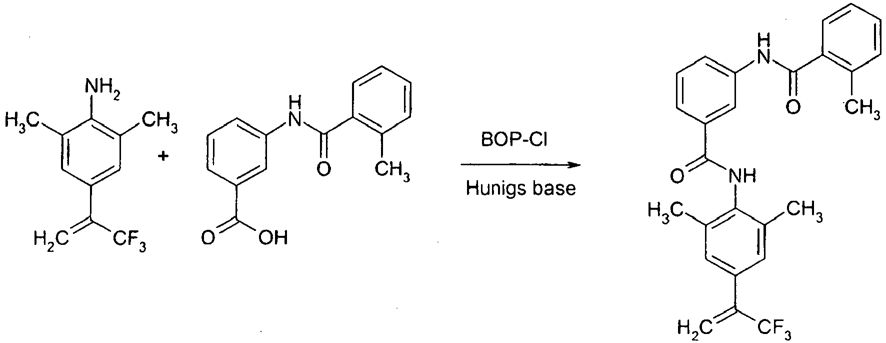

2,6-Dimethyl-4-(1-trifluoromethyl-vinyl)-phenylamine (700 mg, 3.25 mmol) (Example I2), 3-(2-methyl-benzoylamino)-benzoic acid (830 mg, 3.25 mmol) (Example I4), bis(2-oxo-3-oxazolidinyl)phosphonic chloride (BOP-Cl) (994 mg, 3.90 mmol) and N,N-diisopropyl-ethylamine (Hunigs base) (1.67 ml, 9.76 mmol) were dissolved in dimethylformamide (15 ml). The reaction mixture was stirred at 50°C for 18 hours and then allowed to cool to ambient temperature. The reaction mixture was diluted with water (200 ml) and ethyl acetate (200 ml) and the phases were separated. The organic phase was washed twice with water. The water phase was extracted once with ethyl acetate. The organic phases were combined, dried over sodium sulfate and concentrated. Purification by chromatography over silica gel (eluent: hexane / ethyl acetate 4:1 to 1:1) gave 2-methyl-benzoic acid 3-[2,6-dimethyl-4-(1-trifluoromethyl-vinyl)-phenylcarbamoyl]-phenyl amide (378 mg, 26% yield).
¹H-NMR (CDCl₃, 400 MHz): 8.25 (s, 1H), 7.85 (s, 1H), 7.75 (d, 1H), 7.7 (s, 1H), 7.65 (s, 1H), 7.5 (d, 2H), 7.4 (d, 1H), 7.3 (s, 1H), 7.25 (s, 1H), 7.2 (s, 2H), 7.1 (s, 1H), 5.95 (s, 1H), 5.75 (s, 1H), 2.5 (s, 3H), 2.3 (s, 6H).

4-Cyano-benzoic acid 3-[2,6-dimethyl-4-(1-trifluoromethyl-vinyl)-phenylcarbamoyl]-phenyl amide, 2,3-difluoro-benzoic acid 3-[2,6-dimethyl-4-(1-trifluoromethyl-vinyl)-phenylcarbamoyl]-phenyl amide and 4-fluoro-benzoic acid 3-[2,6-dimethyl-4-(1-trifluoromethyl-vinyl)-phenylcarbamoyl]-phenyl amide were made using analogous methods.

### Example P1: Preparation of 2-methyl-benzoic acid 3-{4-(3-(4-fluoro-phenyl)-5-trifluoromethyl-4,5-dihydro-isoxazol-5-yl]-2,6-dimethyl-phenylcarbamoyl}-phenyl amide (Compound A3 of Table A)

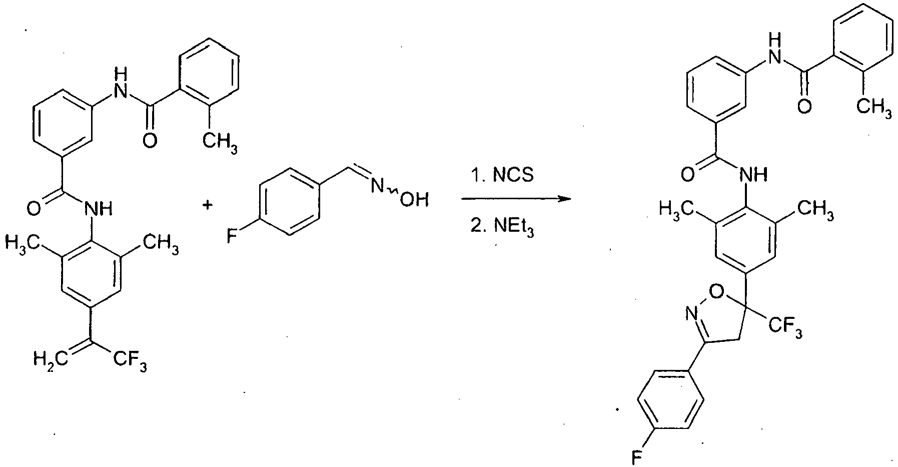

4-Fluoro-benzaldehyde oxime (93 mg, 0.66 mmol) and N-chlorosuccinimide (NCS) (89 mg, 0.66 mmol) were dissolved in dimethylformamide (1 ml). The reaction mixture was stirred at ambient temperature for 90 minutes. A solution of 2-methyl-benzoic acid 3-[2,6-dimethyl-4-(1-trifluoromethyl-vinyl)-phenylcarbamoyl]-phenyl amide (100 mg, 0.22 mmol) (Example 15) and triethylamine (93 µl, 0.66 mmol) in dimethylformamide (1 ml) was added and the reaction mixture was stirred at ambient temperature for 18 hours. The reaction mixture was diluted with water (40 ml) and ethyl acetate (40 ml) and the phases were separated. The organic phase was washed twice with water and the aqueous phases were extracted once with ethyl acetate. The organic phases were combined, dried over sodium sulfate and concentrated. Purification by preparative normal phase HPLC gave Compound A3 of Table A (99 mg, 77% yield).
¹H-NMR (CDCl₃, 400 MHz): 8.25 (s, 1H), 7.85 (d, 1H), 7.65 (m, 3H), 7.6 (s, 1H), 7.5 (d, 2H), 7.4 (s, 1H), 7.35 (s, 2H), 7.3 (s, 1H), 7.1 (t, 2H), 4.05 (d, 1 H), 3.75 (d, 1H), 2.5 (s, 3H), 2.3 (s, 6H).

The compounds A1, A2 and A4-A11 of Table A were made using analogous methods.

### Example I6: Preparation of N-[2,6-dimethyl-4-(1-trifluoromethyl-vinyl)-phenyl]-3-nitro-benzamide

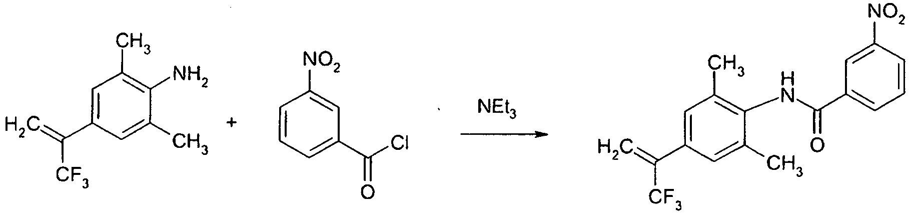

2,6-Dimethyl-4-(1-trifluoromethyl-vinyl)-phenylamine (1.19 g, 5.55 mmol) (Example 12) was dissolved in toluene (5 ml). Triethylamine (1.1 ml, 8.3 mmol) was added. Then at 0°C, a solution of 3-nitro-benzoyl chloride (1.03 g, 5.55 mmol) in toluene (3 ml) was added slowly. The reaction mixture was stirred at 0°C for 2 hours and then at ambient temperature for 2 hours. The crude product was isolated by filtration and purified by preparative normal phase HPLC to give N-[2,6-dimethyl-4-(1-trifluoromethyl-vinyl)-phenyl]-3-nitro-benzamide (1.29 g, 68% yield).
¹H-NMR (CDCl₃, 400 MHz): 8.75 (s, 1H), 8.45 (d, 1H), 8.3 (d, 1H), 7.75 (t, 1H), 7.5 (s, 1H), 7.2 (s, 2H), 6.0 (s, 1H), 5.8 (s, 1H), 2.3 (s, 3H).

### Example I7: Preparation of N- [4-[3-(4-chloro-phenyl)-5-trifluoromethyl-4,5-dihydro-isoxazol-5-yl]-2,6-dimethyl-phenyl}-3-nitrobenzamide

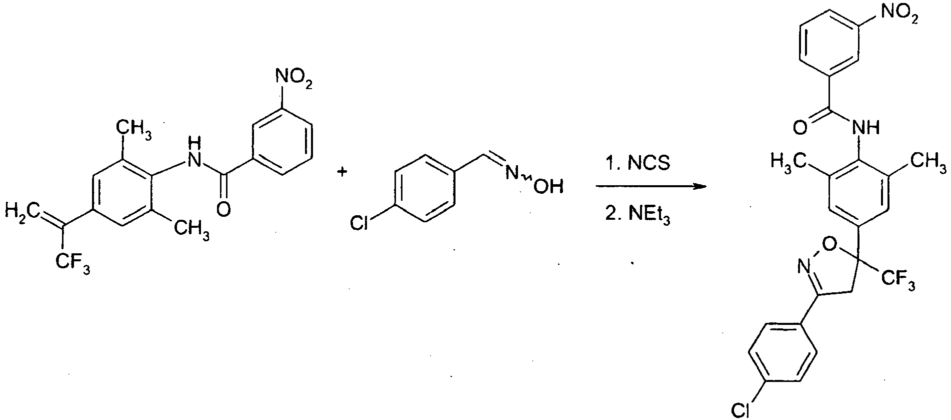

4-Chloro-benzaldehyde oxime (1.93 g, 12.4 mmol) and N-chlorosuccinimide (NCS) (1.66 g, 12.4 mmol) were dissolved in dimethylformamide (10 ml). The reaction mixture was stirred at ambient temperature for 2 hours. A solution of N-[2,6-dimethyl-4-(1-trifluoromethyl-vinyl)-phenyl]-3-nitro-benzamide (1.29 g, 3.55 mmol) (Example 16) and triethylamine (1.73 ml, 3.5 mmol) in dimethylfonnamide (10 ml) was added and the reaction mixture stirred at ambient temperature for 3 hours. The reaction mixture was diluted with water and ethyl acetate and the phases were separated. The organic phase was washed twice with water and the aqueous phases were extracted once with ethyl acetate. The organic phases were combined, dried over sodium sulfate and concentrated. Purification by preparative normal phase HPLC gave N-{4-[3-(4-chloro-phenyl)-5-trifluoromethyl-4,5-dihydro-isoxazol-S-yl]-2,6-dimethyl-phenyl}-3-nitrobenzamide (1.58 g, 86% yield).
¹H-NMR (CDCl₃, 400 MHz): 8.75 (s, 1H), 8.45 (d, 1H), 8.3 (d, 1H), 7.75 (t, 1H), 7.65-7.40(m, 8H), 4.05 (d, 1H), 3.75 (d, 1H), 2.3 (s, 6H).

### Example I8: Preparation of N-{4-[3-(4-chloro-phenyl)-5-trifluoromethyl-4,5-dihydro-isoxazol-5-yl]-2,6-dimethyl-phenyl}-3 -aminobenzamide

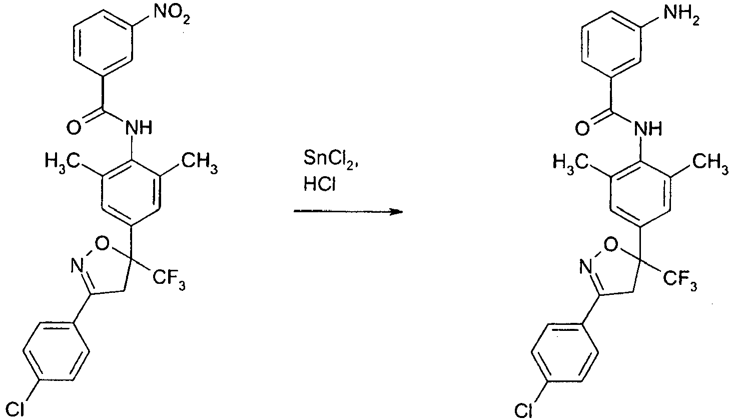

To a solution of N-{4-[3-(4-chloro-phenyl)-5-trifluoromethyl-4,5-dihydro-isoxazol-5-yl]-2,6-dimethyl-phenyl}-3-nitrobenzamide (1.3 g, 2.51mmol) (Example 17) in 2-propanol (20 ml) were added tin(II) chloride (1.72 g, 9.0 mmol) and slowly concentrated hydrochloric acid (2.43 ml). The reaction mixture was stirred at 80°C for 2 hours and then allowed to cool to ambient temperature. The reaction mixture was diluted with water and dichloromethane and the phases were separated. The organic phase was washed twice with water and the water phases were extracted once with dichloromethane. The organic phases were combined, dried over sodium sulfate and concentrated. Crystallisation from ethyl acetate and hexanes gave N-{4-[3-(4-chloro-phenyl)-5-trifluoromethyl-4,5-dihydro-isoxazol-5-yl]-2,6-dimethyl-phenyl}-3-aminobenzamide (1.27 g, 86% yield).
¹H-NMR (CDCl₃, 400 MHz): 7.60-7.20 (m, 13H), 4.05 (d, 1H), 3.75 (d, I H), 2.3 (s, 6H).

### Example P2: Preparation of 4-trifluoromethyl -benzoic acid 3-{4-[3-(4-fluoro-phenyl)-5-trifluoromethyl-4,5-dihydro-isoxazol-5-yl]-2,6-dimethyl-phenylcarbamoyl}-phenyl amide (Compound A30 of Table A)

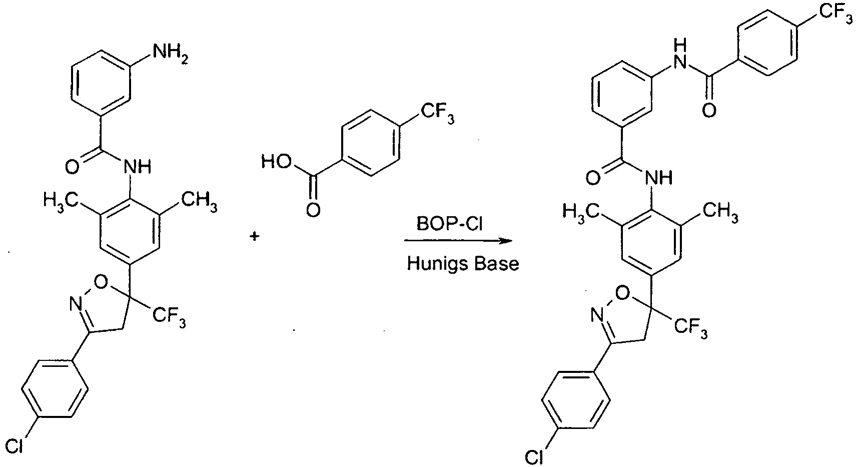

N- {4-[3-(4-Chloro-phenyl)-5-trifluoromethyl-4,5-dihydro-isoxazol-5-yl]-2,6-dimethyl-phenyl}-3-aminobenzamide (50 mg, 0.10 mmol) (Example 18), 4-trifluoromethylbenzoic acid (20 mg, 0.1 mmol), bis(2-oxo-3-oxazolidinyl)phosphonic chloride (BOP-C1) (32 mg, 0.12 mmol) and N,N-diisopropyl-ethylamine (Hunigs base) (53 µl, 0.3 mmol) were dissolved in dimethylformamide (15 ml). The reaction mixture was stirred at ambient temperature for 20 hours. The reaction mixture was diluted with water (20 ml) and ethyl acetate (20 ml) and the phases were separated. The organic phase was washed twice with water. The water phase was extracted once with ethyl acetate. The organic phases were combined, dried over sodium sulfate and concentrated. Purification by preparative normal phase HPLC gave Compound A30 of Table A (55 mg, 82% yield).
¹H-NMR (CDCl₃, 400 MHz): 8.40 (s, 1H), 8.25 (s, 1H), 8.00-7.25 (m, 14H), 4.0 (d, 1H), 3.65 (d, 1H), 2.25 (s, 6H).

The compounds A12-A29 of Table A were made using analogous methods.

The following methods (Method **A** and Method **B**) were used for HPLC-MS analysis:
Method **A** (Agilent 1100 LC) with the following HPLC gradient conditions (Solvent A: 0.05% of formic acid in water and Solvent B: 0.04% of formic acid in acetonitrile / methanol 4:1)

| Time (minutes) | A (%) | B (%) | Flow rate (ml/min) |
|---|---|---|---|
| 0 | 95 | 5 | 1.7 |
| 2.0 | 0 | 100 | 1.7 |
| 2.8 | 0 | 100 | 1.7 |
| 2.9 | 95 | 5 | 1.7 |
| 3.1 | 95 | 5 | 1.7 |

Type of column: Phenomenex Gemini C18; Column length: 30 mm; Internal diameter of column: 3 mm; Particle Size: 3 micron; Temperature: 60°C.
Method **B** (Water Alliance 2795 LC) with the following HPLC gradient conditions (Solvent A: 0.1% formic acid in water / acetonitrile (9: 1) and Solvent B: 0.1 % formic acid in acetonitrile)

| Time (minutes) | A (%) | B (%) | Flow rate (ml/min) |
|---|---|---|---|
| 0 | 90 | 10 | 1.7 |
| 2.5 | 0 | 100 | 1.7 |
| 2.8 | 0 | 100 | 1.7 |
| 2.9 | 950 | 10 | 1.7 |

Type of column: Water atlantis dc18; Column length: 20 mm; Internal diameter of column: 3 mm; Particle Size: 3 micron; Temperature: 40°C.

The characteristic values obtained for each compound were the retention time ("RT", recorded in minutes) and the molecular ion, typically the cation MH⁺ as listed in Table A. The HPLC-MS method used is indicated in brackets.

**Table A: Compounds of formula (Ia):**

| | | | | | | | |
|---|---|---|---|---|---|---|---|
| 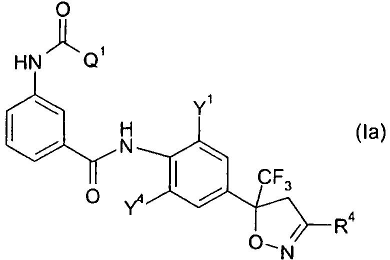 | | | | | | | |

| Compound No. | Q¹ | Y¹ | Y⁴ | R⁴ | RT (min) | MH⁺ | HPLC-MS Method |
|---|---|---|---|---|---|---|---|
| A1 | 4-NC-phenyl- | H | -CH₃ | 4-F-phenyl- | 2.05 | 587 | A |
| A2 | 4-NC-phenyl- | -CH₃ | -CH₃ | 4-F-phenyl- | 2.05 | 601 | A |
| A3 | 2-H₃C-phenyl- | -CH₃ | -CH₃ | 4-F₃C-phenyl- | 2.18 | 640 | A |
| A4 | 2-H₃C-phenyl- | -CH₃ | -CH₃ | 4-F-phenyl- | 2.08 | 590 | A |
| A5 | 2-H₃C-phenyl- | -CH₃ | -CH₃ | 4-Cl-phenyl- | 2.15 | 606 | A |
| A6 | 4-F-phenyl- | -CH₃ | -CH₃ | 4-F-phenyl- | 2.08 | 594 | A |
| A7 | 4-F-phenyl- | -CH₃ | -CH₃ | 4-F₃C-phenyl- | 2.16 | 644 | A |
| A8 | 4-F-phenyl- | -CH₃ | -CH₃ | 4-Cl-phenyl- | 2.17 | 610 | A |
| A9 | 2,3-di-F-phenyl- | -CH₃ | -CH₃ | 4-F-phenyl- | 2.07 | 612 | A |
| A10 | 2,3-di-F-phenyl- | -CH₃ | -CH₃ | 4-F₃C-phenyl- | 2.18 | 662 | A |
| A11 | 2,3-di-F-phenyl- | -CH₃ | -CH₃ | 4-Cl-phenyl- | 2.15 | 628 | A |
| A12 | 2-Cl-pyrid-3-yl- | -CH₃ | -CH₃ | 4-Cl-phenyl- | 1.94 | 627 | B |
| A13 | 2-F-pyrid-3-yl- | -CH₃ | -CH₃ | 4-Cl-phenyl- | 2.00 | 611 | B |
| A14 | 4-NC-phenyl- | -CH₃ | -CH₃ | 4-Cl-phenyl- | 2.10 | 617 | B |
| A15 | 3-Cl-phenyl- | -CH₃ | -CH₃ | 4-Cl-phenyl- | 2.22 | 626 | B |
| A16 | 2-Cl-pyrid-4-yl- | -CH₃ | -CH₃ | 4-Cl-phenyl- | 2.05 | 627 | B |
| A17 | 5-Br-pyrid-3-yl- | -CH₃ | -CH₃ | 4-Cl-phenyl- | 2.06 | 671 | B |
| A18 | 5-Br-furan-2-yl- | -CH₃ | -CH₃ | 4-Cl-phenyl- | 2.10 | 660 | B |
| A19 | 2-Br-phenyl- | -CH₃ | -CH₃ | 4-Cl-phenyl- | 2.10 | 670 | B |
| A20 | 3-Cl-5-F₃C-pyrid-2-yl- | -CH₃ | -CH₃ | 4-Cl-phenyl- | 2.20 | 695 | B |
| A21 | 2-H₃CS-pyrid-3-yl- | -CH₃ | -CH₃ | 4-Cl-phenyl- | 2.00 | 639 | B |
| A22 | 2,5-di-Cl-phenyl- | -CH₃ | -CH₃ | 4-Cl-phenyl- | 2.22 | 660 | B |
| A23 | 6-Cl-pyrid-3-yl- | -CH₃ | -CH₃ | 4-Cl-phenyl- | 2.00 | 627 | B |
| A24 | 4-O₂N-phenyl- | -CH₃ | -CH₃ | 4-Cl-phenyl- | 2.10 | 637 | B |
| A25 | phenyl- | -CH₃ | -CH₃ | 4-Cl-phenyl- | 2.08 | 592 | B |
| A26 | 5-Cl-thiophen-2-yl- | -CH₃ | -CH₃ | 4-Cl-phenyl- | 2.20 | 632 | B |
| A27 | 1,3-di-H₃C-1H-pyrazol-5-yl- | -CH₃ | -CH₃ | 4-Cl-phenyl- | 2.00 | 610 | B |
| A28 | 1,2,3-thiadiazol-4-yl- | -CH₃ | -CH₃ | 4-Cl-phenyl- | 2.00 | 600 | B |
| A29 | 3-H₃C-pyrid-2-yl- | -CH₃ | -CH₃ | 4-Cl-phenyl- | 2.16 | 607 | B |
| A30 | 4-F₃C-phenyl- | -CH₃ | -CH₃ | 4-Cl-phenyl- | 2.23 | 660 | A |

### Biological examples

This Example illustrates the pesticidal/insecticidal properties of compounds of formula (I). Tests were performed as follows:

### Spodopfera littoralis (Egyptian cotton leafworm):

Cotton leaf discs were placed on agar in a 24-well microtiter plate and sprayed with test solutions at an application rate of 200 ppm. After drying, the leaf discs were infested with 5 L1 larvae. The samples were checked for mortality, feeding behaviour, and growth regulation 3 days after treatment (DAT).
The following compounds gave at least 80% control of *Spodoptera littoralis:* A3, A5, A7, A8, A9, A10, A11, A12, A13, A19, A25.

### Heliothis virescens (Tobacco budworm):

Eggs (0-24 h old) were placed in 24-well microtiter plate on artificial diet and treated with test solutions at an application rate of 200 ppm (concentration in well 18 ppm) by pipetting. After an incubation period of 4 days, samples were checked for egg mortality, larval mortality, and growth regulation.
The following compounds gave at least 80% control of *Heliothis virescens:* A1, A2, A3, A4, A5, A6, A7, A8, A9, A10, A11, A12, A13, A14, A15, A16, A17, A18, A19, A20, A21, A22, A23, A24, A25, A26, A27, A28, A29, A30.

### Plutella xylostella (Diamond back moth):

24-well microtiter plate (MTP) with artificial diet was treated with test solutions at an application rate of 200 ppm (concentration in well 18 ppm) by pipetting. After drying, the MTP's were infested with L2 larvae (7-12 per well). After an incubation period of 6 days, samples were checked for larval mortality and growth regulation.
The following compounds gave at least 80% control of *Plutella xylostella:* A3, A4, A5, A6, A7, A8, A9, A10, A11, A12, A13, A14, A15, A16, A 17, A 18, A19, A20, A22, A23, A24, A25, A27, A29.

### Diabrotica balteata (Com root wonn):

A 24-well microtiter plate (MTP) with artificial diet was treated with test solutions at an application rate of 200 ppm (concentration in well 18 ppm) by pipetting. After drying, the MTP's were infested with L2 larvae (6-10 per well). After an incubation period of 5 days, samples were checked for larval mortality and growth regulation.
The following compounds gave at least 80% control of *Diabrotica balteata:* A1, A2, A4, A5, A6, A7, A8, A9, A10, A11, A12, A13, A14, A15, A16, A17, A18, A19, A20, A21, A22, A23, A24, A25, A26, A27, A29, A30.

### Aedes aegypti (Yellow fever mosquito):

10-15 Aedes larvae (L2) together with a nutrition mixture are placed in 96-well microtiter plates. Test solutions at an application rate of 2 ppm were pipetted into the wells. 2 days later, insects were checked for mortality and growth inhibition.
The following compounds gave at least 80% control of *Aedes aegypti:* A1, A2, A3, A4, A5, A6, A7, A8, A9, A10, A11, A12, A13, A14, A15, A16, A17, A18, A23, A24, A25.

## Claims

1. A compound of formula (I): 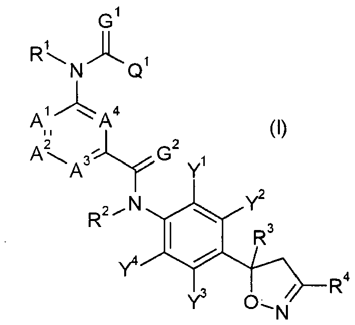 wherein
A¹, A², A³ and A⁴ are C-X;
each X is independently hydrogen, halogen, C₁-C₄alkyl, C₁-C₄haloalkyl or C₁-C₄alkoxy; R¹ and R² are hydrogen;
G¹ and G² are oxygen;
Q¹ is phenyl or phenyl substituted by one to five substituents R⁵, which may be the same or different, or Q¹ is heterocyclyl or heterocyclyl substituted by one to five substituents R⁵, which may be the same or different;
R³ is trifluoromethyl;
R⁴ is phenyl or phenyl substituted by one to five substituents R⁵, which may be the same or different;
Y¹ and Y⁴ are independently of each other hydrogen, cyano, halogen, C₁-C₄alkyl or C₁-C₄haloalkyl;
Y² and Y³ are independently of each other hydrogen, halogen or C₁-C₄alkyl; and
each R⁵ is independently cyano, nitro, hydroxy, halogen, C₁-C₄alkyl, C₁-C₄haloalkyl, C₁-C₃alkoxy or C₁-C₃alkylthio ;
wherein said heterocyclyl is selected from pyridyl, pyridazinyl, pyrimidinyl, pyrazinyl, furanyl, thiophenyl, oxazolyl, isoxazolyl, oxadiazolyl, thiazolyl, isothiazolyl, thiadiazolyl, pyrrolyl, pyrazolyl, imidazolyl, triazolyl and tetrazolyl;
or salts or N-oxides thereof.

2. A compound according to claim 1 wherein each X is independently hydrogen, fluoro, chloro, bromo, methyl, trifluoromethyl or methoxy.

3. A compound according to either claim 1 or claim 2 wherein Q¹ is phenyl, pyridyl, furanyl, thiophenyl, pyrazolyl or 1,2,3-thiadiazolyl, or phenyl, pyridyl, furanyl, thiophenyl, pyrazolyl or 1,2,3-thiadiazolyl substituted by one to three substituents independently selected from cyano, nitro, hydroxy, fluoro, chloro, bromo, methyl, trifluoromethyl, methoxy and methylthio.

4. A compound according to any one of claims 1 to 3 wherein R⁴ is phenyl or phenyl substituted by one to five substituents selected from halogen, C₁-C₄alkyl or C₁-C₄haloalkyl.

5. A compound according to any one of claims 1 to 4 wherein Y¹ is cyano, chloro, methyl, ethyl or trifluoromethyl.

6. A compound according to any one of claims 1 to 5 wherein Y² is hydrogen, fluoro, chloro or methyl.

7. A compound according to any one of claims 1 to 6 wherein Y³ is hydrogen, fluoro, chloro or methyl.

8. A compound according to any one of claims 1 to 7 wherein Y⁴ is cyano, chloro, methyl, ethyl or trifluoromethyl.

9. A compound of formula (II) 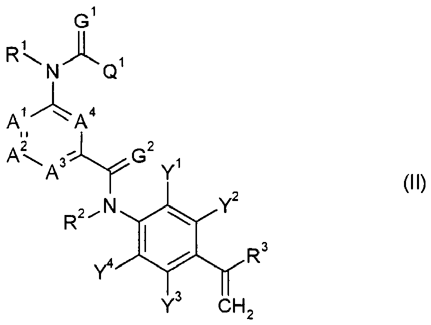 wherein A¹, A², A³, A⁴, R¹, R², R³, G¹, G², Q¹, Y¹, Y², Y³ and Y⁴ are as defined in claim 1; or salts or N-oxides thereof;
a compound of formula (XII) 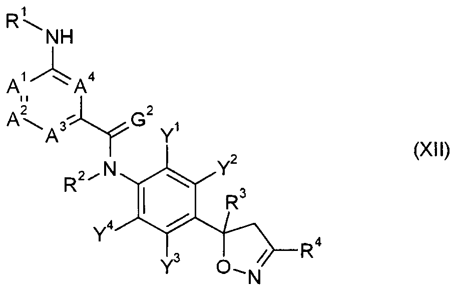 wherein A¹, A², A³, A⁴, R¹, R², R³, R⁴, G², Y¹, Y², Y³ and Y⁴ are as defined in claim 1; or salts or N-oxides thereof;
a compound of formula (XIII) 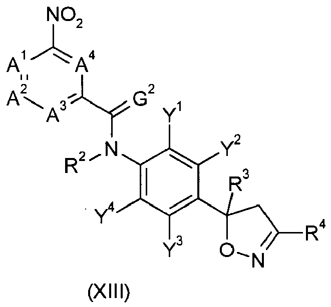 wherein A¹, A², A³, A⁴, R², R³, R⁴, G², Y¹, Y², Y³ and Y⁴ are as defined in claim 1; or salts or N-oxides thereof;
a compound of formula (XIV) 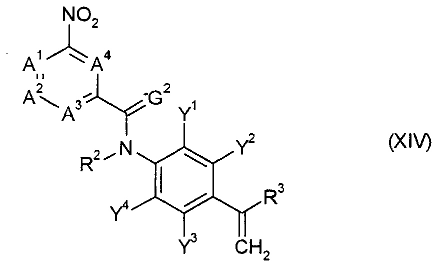 wherein A¹, A², A³, A⁴, R², R³, G², Y¹, Y², Y³ and Y⁴ are as defined in claim 1; or salts or N-oxides thereof.

10. A method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I) as defined in any one of claims 1 to 8 with the exception of a method for treatment of the human or animal body by surgery or therapy and diagnostic methods practised on the human or animal body.

11. An insecticidal, acaricidal, nematicidal or molluscicidal composition comprising an insecticidally, acaricidally, nematicidally or molluscicidally effective amount of a compound of formula (I) as defined in any one of claims 1 to 8.

## Patentansprüche

1. Verbindung der Formel (I): 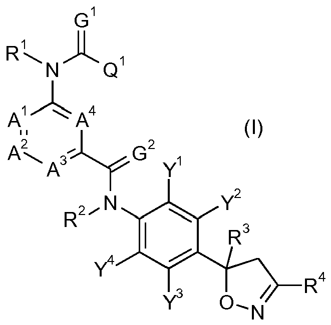 in der
A¹, A², A³ und A⁴ C-X bedeuten;
X jeweils unabhängig Wasserstoff, Halogen, C₁-C₄-Alkyl, C₁-C₄-Halogenalkyl oder C₁-C₄-Alkoxy bedeutet;
R¹ und R² Wasserstoff bedeuten;
G¹ und G² Sauerstoff bedeuten;
Q¹ Phenyl oder Phenyl, das durch ein bis fünf Substituenten R⁵, die gleich oder verschieden sein können, substituiert ist, bedeutet, oder Q¹ Heterocyclyl oder Heterocyclyl, das durch einen bis fünf Substituenten R⁵, die gleich oder verschieden sein können, substituiert ist, bedeutet,
R³ Trifluormethyl bedeutet;
R⁴ Phenyl oder Phenyl, das durch ein bis fünf Substituenten R⁵, die gleich oder verschieden sein können, substituiert ist, bedeutet;
Y¹ und Y⁴ unabhängig voneinander Wasserstoff, Cyano, Halogen oder C₁-C₄-Alkyl oder C₁-C₄-Halogenalkyl bedeuten;
Y² und Y³ unabhängig voneinander Wasserstoff, Halogen oder C₁-C₄-Alkyl bedeuten; und
R⁵ jeweils unabhängig Cyano, Nitro, Hydroxy, Halogen, C₁-C₄-Alkyl, C₁-C₄-Halogenalkyl, C₁-C₃-Alkoxy oder C₁-C₃-Alkylthio bedeutet;
wobei das Heterocyclyl aus der Reihe Pyridyl, Pyridazinyl, Pyrimidinyl, Pyrazinyl, Furanyl, Thiophenyl, Oxazolyl, Isoxazolyl, Oxadiazolyl, Thiazolyl, Isothiazolyl, Thiadiazolyl, Pyrrolyl, Pyrazolyl, Imidazolyl, Trizolyl und Tetrazolyl ausgewählt ist;
oder Salze oder N-Oxide davon.

2. Verbindung nach Anspruch 1, worin X jeweils unabhängig Wasserstoff, Fluor, Chlor, Brom, Methyl, Trifluormethyl oder Methoxy bedeutet.

3. Verbindung nach Anspruch 1 oder Anspruch 2, worin Q¹ Phenyl, Pyridyl, Furanyl, Thiophenyl, Pyrazolyl oder 1,2,3-Thiadiazolyl bedeutet oder Phenyl, Pyridyl, Furanyl, Thiophenyl, Pyrazolyl oder 1,2,3-Thiadiazolyl, das jeweils durch einen bis drei Substituenten, die unabhängig aus der Reihe Cyano, Nitro, Hydroxy, Fluor, Chlor, Brom, Methyl, Trifluormethyl, Methoxy und Methylthio ausgewählt sind, substituiert ist, bedeutet.

4. Verbindung nach einem der Ansprüche 1 bis 3, worin R⁴ Phenyl oder Phenyl, das durch einen bis fünf Substituenten, die aus der Reihe Halogen, C₁-C₄-Alkyl oder C₁-C₄-Halogenalkyl ausgewählt sind, substituiert ist, bedeutet.

5. Verbindung nach einem der Ansprüche 1 bis 4, worin Y¹ Cyano, Chlor, Methyl, Ethyl oder Trifluormethyl bedeutet.

6. Verbindung nach einem der Ansprüche 1 bis 5, worin Y² Wasserstoff, Fluor, Chlor oder Methyl bedeutet.

7. Verbindung nach einem der Ansprüche 1 bis 6, worin Y³ Wasserstoff, Fluor, Chlor oder Methyl bedeutet.

8. Verbindung nach einem der Ansprüche 1 bis 7, worin Y⁴ Cyano, Chlor, Methyl, Ethyl oder Trifluormethyl bedeutet.

9. Verbindung der Formel (II), 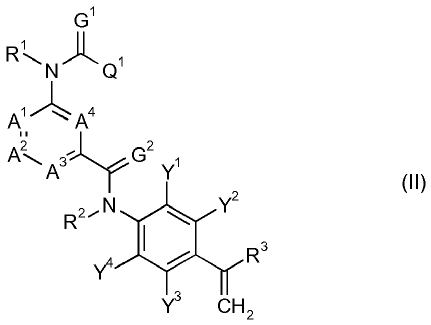 worin A¹, A², A³, A⁴, R¹, R², R³, G¹, G², Q¹, Y¹, Y², Y³ und Y⁴ wie in Anspruch 1 definiert sind; oder Salze oder N-Oxide davon;
Verbindung der Formel (XII), 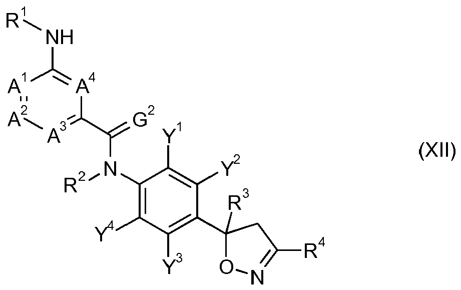 worin A¹, A², A³, A⁴, R¹, R², R³, R⁴, G², Y¹, Y², Y³ und Y⁴ wie in Anspruch 1 definiert sind; oder Salze oder N-Oxide davon;
Verbindung der Formel (XIII), 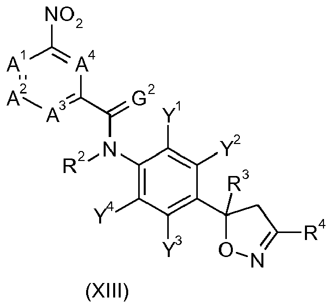 worin A¹, A², A³, A⁴, R², R³, R⁴, G², Y¹, Y², Y³ und Y⁴ wie in Anspruch 1 definiert sind; oder Salze oder N-Oxide davon;
Verbindung der Formel (XIV), 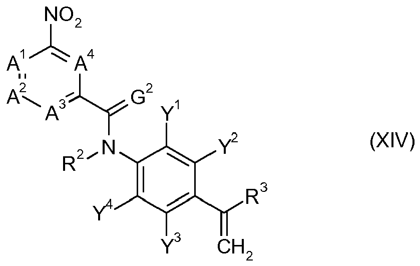 worin A¹, A², A³, A⁴, R², R³, G², Y¹, Y², Y³ und Y⁴ wie in Anspruch 1 definiert sind; oder Salze oder
N-Oxide davon.

10. Verfahren zum Bekämpfen und Kontrollieren von Insekten, Acarina, Nematoden oder Mollusken, bei dem man eine insektizid, akarizid, nematizid oder molluskizid wirksame Menge einer Verbindung der Formel (I) wie in einem der Ansprüche 1 bis 8 definiert auf einen Schädling, den Ort eines Schädlings oder auf eine Pflanze, die von einem Schädling befallen werden kann, appliziert, mit Ausnahme eines Verfahrens für die Behandlung des menschlichen oder tierischen Körpers mittels Chirurgie oder Therapie und diagnostischen Verfahren, die an dem menschlichen oder tierischen Körper durchgeführt werden.

11. Insektizide, akarizide, nematizide oder molluskizide Zusammensetzung, die eine insektizid, akarizid, nematizid oder molluskizid wirksame Menge einer Verbindung der Formel I wie in einem der Ansprüche 1 bis 8 definiert umfasst.

## Revendications

1. Composé de formule (I) : 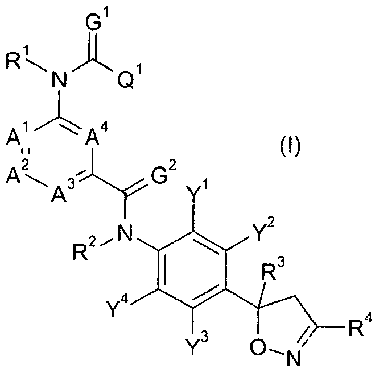 dans laquelle
A¹, A², A³ et A⁴ sont C-X ;
chaque X est indépendamment hydrogène, halogène, C₁-C₄alkyle, C₁-C₄halogénoalkyle ou C₁-C₄alcoxy ;
R¹ et R² sont hydrogène ;
G¹ et G² sont oxygène ;
Q¹ est phényle ou phényle substitué par de un à cinq substituants R⁵, qui peuvent être identiques ou différents, ou Q¹ est hétérocyclyle ou hétérocyclyle substitué par de un à cinq substituants R⁵, qui peuvent être identiques ou différents ;
R³ est trifluorométhyle ;
R⁴ est phényle ou phényle substitué par de un à cinq substituants R⁵, qui peuvent être identiques ou différents ;
Y¹ et Y⁴ sont indépendamment l'un de l'autre hydrogène, cyano, halogène, C₁-C₄alkyle ou C₁-C₄halogénoalkyle ;
Y² et Y³ sont indépendamment l'un de l'autre hydrogène, halogène ou C₁-C₄alkyle ; et
chaque R⁵ est indépendamment cyano, nitro, hydroxy, halogène, C₁-C₄alkyle, C₁-C₄halogénoalkyle, C₁-C₃alcoxy ou C₁-C₃alkylthio ;
où ledit hétérocyclyle est choisi parmi pyridyle, pyridazinyle, pyrimidinyle, pyrazinyle, furanyle, thiophényle, oxazolyle, isoxazolyle, oxadiazolyle, thiazolyle, isothiazolyle, thiadiazolyle, pyrrolyle, pyrazolyle, imidazolyle, triazolyle et tétrazolyle ;
ou les sels ou N-oxydes de celui-ci.

2. Composé selon la revendication 1, dans lequel chaque X est indépendamment hydrogène, fluoro, chloro, bromo, méthyle, trifluorométhyle ou méthoxy.

3. Composé selon la revendication 1 ou bien la revendication 2, dans lequel Q¹ est phényle, pyridyle, furanyle, thiophényle, pyrazolyle ou 1,2,3-thiadiazolyle, ou phényle, pyridyle, furanyle, thiophényle, pyrazolyle ou 1,2,3-thiadiazolyle substitué par de un à trois substituants choisis indépendamment parmi cyano, nitro, hydroxy, fluoro, chloro, bromo, méthyle, trifluorométhyle, méthoxy et méthylthio.

4. Composé selon l'une quelconque des revendications 1 à 3, dans lequel R⁴ est phényle ou phényle substitué par de un à cinq substituants choisis parmi halogène, C₁-C₄alkyle ou C₁-C₄halogénoalkyle.

5. Composé selon l'une quelconque des revendications 1 à 4, dans lequel Y¹ est cyano, chloro, méthyle, éthyle ou trifluorométhyle.

6. Composé selon l'une quelconque des revendications 1 à 5, dans lequel Y² est hydrogène, fluoro, chloro ou méthyle.

7. Composé selon l'une quelconque des revendications 1 à 6, dans lequel Y³ est hydrogène, fluoro, chloro ou méthyle.

8. Composé selon l'une quelconque des revendications 1 à 7, dans lequel Y⁴ est cyano, chloro, méthyle, éthyle ou trifluorométhyle.

9. Composé de formule (II) 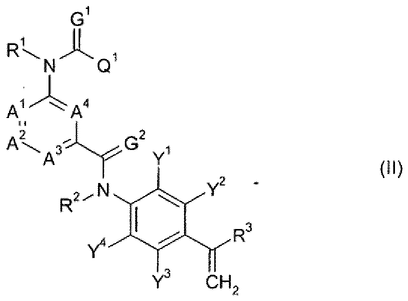 dans laquelle A¹, A², A³, A⁴, R¹, R², R³, G¹, G², Q¹, Y¹, Y², Y³ et Y⁴ sont tels que définis selon la revendication 1 ; ou les sels ou N-oxydes de celui-ci ; un composé de formule (XII) 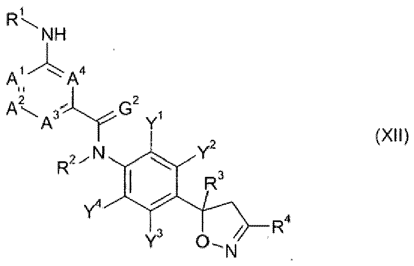 dans laquelle A¹, A², A³, A⁴, R¹, R², R³, R⁴, G², Y¹, Y², Y³ et Y⁴ sont tels que définis selon la revendication 1 ; ou les sels ou N-oxydes de celui-ci ;
un composé de formule (XIII) 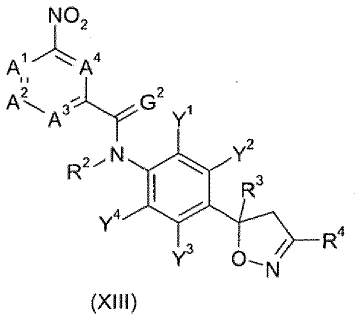 dans laquelle A¹, A², A³, A⁴, R², R³, R⁴, G², Y¹, Y², Y³ et Y⁴ sont tels que définis selon la revendication 1 ; ou les sels ou N-oxydes de celui-ci ;
un composé de formule (XIV) 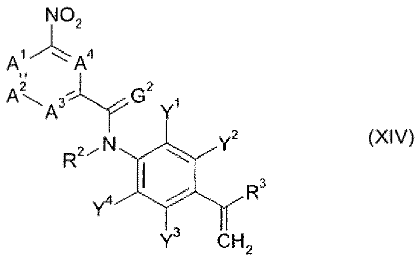 dans laquelle A¹, A², A³, A⁴, R², R³, G², Y¹, Y², Y³ et Y⁴ sont tels que définis selon la revendication 1 ; ou les sels ou N-oxydes de celui-ci.

10. Méthode de lutte et de contrôle d'insectes, d'acariens, de nématodes ou de mollusques, comprenant l'application à un organisme nuisible, au lieu où se développe l'organisme nuisible, ou à une plante susceptible d'être attaquée par un organisme nuisible, d'une quantité efficace sur le plan insecticide, acaricide, nématicide ou molluscicide d'un composé de formule (I) tel que défini selon l'une quelconque des revendications 1 à 8, à l'exception d'une méthode de traitement d'un corps humain ou animal par chirurgie ou thérapie et des méthodes de diagnostic pratiquées sur le corps humain ou animal.

11. Composition insecticide, acaricide, nématicide ou molluscicide comprenant une quantité efficace sur le plan insecticide, acaricide, nématicide ou molluscicide d'un composé de formule (I) tel que défini selon l'une quelconque des revendications 1 à 8.
